# EUROPEAN PATENT SPECIFICATION

(11) **EP 1 908 777 B1**
(45) Date of publication and mention of the grant of the patent: **03.02.2016**
(21) Application number: 07291219.9
(22) Date of filing: 08.10.2007
(51) Int. Cl.: C07K 14/435, C12N 15/00, A61K 38/17

(54) **Mite fusion proteins**
Milbenfusionsproteine
Protéines de fusion d'acarien

(30) Priority: 06.10.2006 EP 06291561
(43) Date of publication of application: 09.04.2008
(73) Proprietor: STALLERGENES, 92160 Antony (FR)
(72) Inventor: Moingeon, Philippe, 92160 Antony (FR); Bulder, Ingrid, 1057 CG Amsterdam (NL); Bordas, Véronique, 92160 Antony (FR); Bussieres, Laetitia, 78000 Versailles (FR)
(74) Representative: Colombet, Alain André

(56) References cited:
- EP-B1- 1 373 510
- EP-B1- 2 207 795
- WO-A-2004/005334
- BEST E A ET AL: "A Recombinant Group 1 House Dust Mite Allergen, rDer f 1, with Biological Activities Similar to Those of the Native Allergen" PROTEIN EXPRESSION AND PURIFICATION, ACADEMIC PRESS, SAN DIEGO, CA, US, vol. 20, no. 3, December 2000 (2000-12), pages 462-471, XP004435461 ISSN: 1046-5928
- THOMAS W R ET AL: "CLONING AND EXPRESSION OF DNA CODING FOR THE MAJOR HOUSE DUST MITE ALLERGEN DER P 1 IN ESCHERICHIA COLI" INTERNATIONAL ARCHIVES OF ALLERGY AND APPLIED IMMUNOLOGY, BASEL, CH, vol. 85, January 1988 (1988-01), pages 127-129, XP002021266 ISSN: 0020-5915
- HAKKAART G A J ET AL: "Expression of the house dust mite allergen Der p 2 in the baker's yeast Saccharomyces cerevisiae" CLINICAL AND EXPERIMENTAL ALLERGY, BLACKWELL SCIENTIFIC PUBLICATIONS, LONDON, GB, vol. 28, no. 1, January 1998 (1998-01), pages 45-52, XP002297323 ISSN: 0954-7894
- HAKKAART G A J ET AL: "Involvement of the N-terminus of Der p 2 in IgE and monoclonal antibody binding" INTERNATIONAL ARCHIVES OF ALLERGY AND IMMUNOLOGY 1998 SWITZERLAND, vol. 115, no. 2, 1998, pages 150-156, XP009079784 ISSN: 1018-2438
- CHAPMAN MARTIN D ET AL: "Nomenclature and structural biology of allergens.", THE JOURNAL OF ALLERGY AND CLINICAL IMMUNOLOGY FEB 2007, vol. 119, no. 2, February 2007 (2007-02), pages 414-420, ISSN: 0091-6749
- KING T P ET AL: "Allergen nomenclature.", ALLERGY SEP 1995, vol. 50, no. 9, September 1995 (1995-09), pages 765-774, ISSN: 0105-4538

## Description

The invention relates to a fusion protein, an isolated nucleic acid coding for it and its use for the manufacture of a medicament to prevent or treat a mite allergic reaction.

The contribution of mites to allergy in humans was first documented in the 1920s when Cooke and Kern found that dust from vacuum cleaner bags caused positive skin reactions in asthma patients. Later, Voorhorst and colleagues were the first to implicate mites found in dust in the development of dust allergy. These investigators conducted a survey of the European and American continents and discovered they all had astigmatid mites in the genus *Dermatophagoides.* The concentration of mites found in survey samples from carpets and bedding varied from 1 to 500 mites per gram of dust. Mite extracts produced positive skin reactions similar to those observed by Cooke and Kern 43 years earlier. This finding led to the conclusion that *Dermatophagoides* were one of the principal sources of house dust allergens. Later studies confirmed *Dermatophagoides pteronyssinus* to be the most abundant mite species worldwide.

Dust mite allergies are often characterised by 'perennial' or 'year-round' hayfever or allergic rhinitis: frequent sneezing, a runny, stuffy, itchy nose and irritated eyes. Asthma and eczema can also be triggered by a dust mite allergy.

The allergens produced by the genus *Dermatophagoides* mites fall mainly into two immunologically important groups: group I (*Der p 1, Der f* 1) and II (*Der p 2, Der f 2).* Der p 1 and Der p 2 are the major European house dust mite (HDM) allergens from *Dermatophagoides pteronyssinus.* Der f 1 and Der f 2 are the major allergens from *Dermatophagoides farinae.* Group I allergens are approximately 25,000 Da glycoproteins found primarily in mite feces. The Der p 1 allergen occurs at 10mg/mL (0.2ng per fecal pellet) in mite feces and elutes rapidly from fecal pellets. *Der p 1* is a cysteine protease secreted by mites during digestion and is released in fecal pellets that are 10µm to 40µm in size. Group I allergens are structurally homologous (among house dust mites, most particularly *Dermatophagoides pteronyssinus* and *Dermatophagoides farinae* species). Group II allergens, found in mite bodies, are approximately 15,000 Da proteins, and like group I allergens, share structural homologies. Cross-reactivity between species-specific allergens is seen within each group, but is not documented across groups.

Chapman MD et al. (J Immunol. 1980 Aug;125(2):587-92) have described the purification of the Der p 1 protein from *D. pteronyssinus* in 1980.

Group I and II mite antigens are the most clinically relevant to asthma, atopic dermatitis, and allergic rhinitis since more than 80% HDM (house dust mite) allergic patients exhibit high seric IgE titers directed to these two allergens. Currently, at least twenty one different allergen groups have been identified for the European dust mite.

The therapeutic options fall in three major categories. The first opportunity is allergen avoidance or reduction of the exposure. It may be difficult or expensive for house dust mite allergens. The second and most widely used therapeutic option is the prescription of classical symptomatic drugs like anti-histamines and steroids. Symptomatic drugs are safe and efficient; however, they do not alter the natural cause of the disease. The third therapeutic alternative is specific allergy vaccination which, in most cases, reduces or alleviates the allergic symptoms caused by the allergen in question. Conventional specific allergy vaccination is a causal treatment for allergic disease. It interferes with basic immunological mechanisms resulting in persistent improvement of the patients' immune status. Thus, the protective effect of specific allergy vaccination extends beyond the treatment period in contrast to symptomatic drug treatment. Some patients receiving the treatment are cured, and, in addition, most patients experience a relief in disease severity and symptoms experienced, or at least an arrest in disease aggravation. Thus, specific allergy vaccination has preventive effects reducing the risk of rhinitis developing into asthma, and reducing the risk of developing new sensitivities.

The immunological mechanism underlying successful allergy vaccination is not known in detail. It is generally accepted that an active vaccine must have the capacity to stimulate allergen specific T-cells, preferably TH1 and regulatory T cells.
Specific allergy vaccination is, in spite of its virtues, not in widespread use, primarily for two reasons. One reason is the inconveniences associated with the traditional vaccination programme that comprises repeated vaccinations i.e. injections over several months. The other reason is, more importantly, the risk of allergic side reactions. Ordinary vaccinations against infectious agents are efficiently performed using a single or a few high dose immunizations. This strategy, however, cannot be used for allergy vaccination since a pathological immune response is already ongoing. Conventional specific allergy vaccination is therefore carried out using multiple subcutaneous or mucosal (e.g. sublingual) immunizations applied over an extended time period.

Recombinant allergens are an alternative to the complex biological extracts used in specific allergy vaccination with a better yield. Their biological properties as immunogenicity and harmlessness depend on the chosen expression system.

WO 88/10297 discloses the cDNA and amino acid sequences of the mature excreted Der p 1, the transient pre- and preproenzymes forms of Der p 1, and Der p 2. The nucleotide sequence of the cDNA insert of a phage clone reacted with the rabbit anti-Der p 1 serum results in the expression of a fusion protein comprising Der p 1 and a β-galactosidase. This recombinant chimera has been shown to inhibit IgE responses of mice to mature Der p 1.

Recombinant Der p 1 has been expressed in *Pichia pastoris* (Jacquet el al. Clin Exp Allergy. 2002 Jul;32(7):1048-53.) as an hyperglycosylated protein with similar IgE binding and enzymatic activity but decreased histamine realising capacity as compared with the natural allergen.

Expression of Der p 2 in the baker's yeast yields a recombinant protein with properties similar to the naturel protein (Hakkaart GA, Harmsen MM, Chua KY, Thomas WR, Aalberse RC, Van Ree R. Clin Exp Allergy. 1998 Jan;28(1):45-52.).

Recombinant Der f 1 has been produced in insect cells, using a baculovirus expression system (Shoji H. et al., Biosci Biotechnol Biochem. 1996 Apr;60(4):621-5; Shoji H. et al., Biosci Biotechnol Biochem. 1997 Oct;61(10):1668-73) and in *Aspergillus oryzae* (Shoji H. et al., Biosci Biotechnol Biochem. 1999 Apr;63(4):703-9).

Der f 2 has been expressed in *E. coli* (Iwamoto M. et al., Int Arch Allergy Immunol: 1996 Apr;109(4):356-61).

Best et al (2000. Prot Exp Purif, 20(3), 462-471) disclose the production of Der f 1 fusion with a standard tag, namely a pre-pro sequence of factor α of *S*. *Cerevisiae,* to ensure secretion of the recombinant protein. The recombinant protein is recognised by IgE antibodies from allergic patients.

The Applicant has cloned proDer p 1 in tobacco plants (WO2004/005334) and thus expressed four polypeptides recognized by a specific anti-Der p 1 antibody. Three of them are synthesis intermediate species of the mature Der p 1 protein and the fourth one has five amino acids more than the mature Der p 1 protein. It is also shown that the propeptide in proDer p 1 is essential for the expression of a recombinant Der p 1 in a eukaryotic cell (WO 2004/005334). Expression in various prokaryotic and eukaryotic systems (*E. coli,* yeast, CHO, Drosophila cells) have established that the propeptide is necessary for the expression of a well folded molecule : more specifically, expression of mature Der p 1 (i.e. without the propeptide) results in low levels of expression of a Der p 1 molecule which is incorrectly folded.

To replace biological extracts, there is a need for a vaccine product combining several recombinant house dust mite allergens, with a high purity, preferably with natural conformation, with a conserved antigenicity and immunogenicity (both in terms of IgE, IgG, and T lymphocyte recognition) as compared with the natural allergens, and which can be produced in prokaryotic and/or eukaryotic systems.

This is achieved by a fusion protein comprising a Group I allergen and a Group II allergen from the genus *Dermatophagoides,* the allergens having in the fusion protein a conformation close to the one of the natural mature allergens.

The present Inventors have expressed four fusion proteins assembling the two major allergens Der p 1 and Der p 2 from the common house dust mite *Dermatophagoides pteronyssinus.* Der p 2 has been fused at the N-or C-terminal end of either mature Der p 1 or proDer p 1. These four fusion proteins have been successfully expressed in *Escherichia coli.* The two forms with the Der p 1 propeptide have also been expressed in the yeast *Pichia pastoris.* These 6 fusion proteins are recognized in immunoblot analysis by anti-Der p 2 monoclonal antibodies and by IgE from house dust mite allergic patients. The inventors also observed some reactivity with a mouse monoclonal antibody and sheep polyclonal antibodies specific for native Der p 1. Immunoreactivity data confirm that both Der p 1 and Der p 2 are correctly folded in those fusion proteins since some conformational epitopes can be recognized by various antibodies directed to the distinct components of the fusion proteins.

Hence, these results support that a fusion protein can be successfully prepared by fusioning directly together at least two allergens while retaining the folding and immunogenicity of the native antigens.

### Fusion Protein

Thus, the present invention relates to a fusion protein comprising a Group I allergen and a Group II allergen from the genus *Dermatophagoides*,
wherein said Group I allergen from the genus Dermatophagoides is selected from the group consisting of Der p 1, Der f 1, a proform of Der p 1, a proform of Deer f 1, and a sequence at least 90% identical to SEQ ID NO:5 or SEQ ID NO:7 which substantially retains the immunogenicity of the polypeptide of sequence SEQ ID NO:5 or SEQ ID NO:7, respectively; and
wherein said Group II allergen from the genus Dermatophagoides is selected from the group consisting of Der p 2, Der f 2, a proform of Der p 2, a proform of Der f 2, and a sequence at least 90% identical to SEQ ID NO:4 which substantially retains the immunogenicity of the polypeptide of sequence SEQ ID NO:4.

As used herein, a "protein" or "peptide" denotes a molecule comprised of a linear array of amino acid residues connected to each other in the linear array by peptide bonds.

A "fusion protein" refers to a protein having at least a Group I allergen and a Group II allergen from the genus *Dermatophagoides* covalently linked, either directly or via a linker peptide. The polypeptides forming the fusion protein are typically linked C-terminus to N-terminus, although they can also be linked C-terminus to C-terminus, N-terminus to N-terminus, or N-terminus to C-terminus. The polypeptides of the fusion protein can be in any order. Alternatively, the fusion protein could be obtained by chemical coupling.

A linker peptide sequence may be employed to separate the two polypeptide components by a distance sufficient to ensure that each polypeptide folds into its secondary and tertiary structures. This linker sequence may generally be from 1 to about 50 amino acids in length. Linker sequences are not required when the second polypeptide has a dispensable N-terminal region that can be used to separate the functional domains and prevent steric hindrance.

However, in the frame of the present invention, the Group I allergen and the Group II allergen of the fusion protein are preferably directly linked, in order to preclude any risk of immunological reaction to the linker itself.

Accordingly, the Group II allergen is preferably fused directly at the N- or C-terminal end of the Group I allergen. According to one embodiment of the present invention, the fusion protein consists in a N-or C-terminal fusion of a Group I allergen and group II allergen from the genus *Dermatophagoides.*

An "allergen" is defined as a substance, usually a peptide or protein, which elicits the production of IgE antibodies in predisposed individuals. Similar definitions are presented in the following references: Clin. Exp. Allergy, No. 26, pp. 494-516 (1996); Mol. Biol. of Allergy and Immunology, ed. R. Bush, Immunology and Allergy Clinics of North American Series (August 1996).

A "Group I allergen from the genus *Dermatophagoides*" is a peptide or protein retaining substantially the immunogenicity of a natural Group I allergen from the genus *Dermatophagoides.* Preferably, the Group I allergen is Der p 1 or Der f 1, or a proform thereof.

A "Group II allergen from the genus *Dermatophagoides*" is a protein retaining substantially the immunogenicity of a natural Group II allergen from the genus *Dermatophagoides.* Preferably, the Group II allergen is Der p 2 or Der f 2, or a proform thereof.

A "proform" means a precursor of the mature protein, the mature protein being generally obtained by enzymatic processing of the proform. A proform includes preproteins (with a leader peptide), proproteins (with a propeptide) and preproproteins, for example proDer p 1 and preproDer p 1.

"Retaining substantially the immunogenicity of a natural allergen" means retaining completely or partially the ability to induce and react with IgE and IgG antibodies directed towards said natural allergen. It includes in particular allergens with reduced IgE reactivity (hypoallergenic forms).

Consequently, a "Group I (or II) allergen from the genus *Dermatophagoides*" includes peptides or proteins which comprise or consist of the sequence of a natural Group I (or II) allergen, variants, mutants, fragments, especially those encoding selected B and/or T epitopes, as well as homologs thereof with a percentage of identity above 80%, preferably 90%, still preferably 95%, provided that they substantially retain the immunogenicity of a natural Group I (or II) allergen from the genus *Dermatophagoides.*

In one embodiment according to the invention, the Group II allergen is Der p 2 (SEQ ID NO:4) and the Group I allergen is Der p 1 (SEQ ID NO:5) or proDer p 1 (SEQ ID NO:7). Preferably, the fusion protein according to the invention comprises or consists in SEQ ID NO:8 (Der p 2-Der p 1 fusion), SEQ ID NO:9 (Der p 2-ProDer p 1 fusion), SEQ ID NO:10 (Der p 1-Der p 2 fusion), SEQ ID NO:11 (ProDer p 1-Der p 2 fusion). These four fusion proteins are here referred as F1, F2, F3 and F4 respectively.

In another embodiment, the Group II allergen consists in Der f 2 and the Group I allergen is Der f 1 or proDer f 1.

As used herein, a variant sequence is a naturally occurring sequence but which diverges from the reference sequence by some point mutations.

As used herein, a mutant sequence is a sequence of a natural allergen in which one or several mutations have been introduced, for example to abrogate N-glycosylation sites, increase or decrease the cysteine protease activity of said allergen, improve the expression of the protein in an appropriate expression system, for example in *E. coli,* notably taking into account the codon bias, or improve T-cell epitopes anchoring sites so as to enhance the association with MHC class I or class II. Preferred point mutations of the Der p 1 allergen are mutations of the N-glycosylation sites (N34Q and/or N150Q in SEQ ID NO:6) or of the catalytic site (C132V in SEQ ID NO:6).

A fusion protein according to the invention may further comprise at least one additional allergen from the genus *Dermatophagoides.* For example, it may comprise a Group V and/or a Group VII and/or a Group XIII allergen from the genus *Dermatophagoides*, preferably Der p 5, Der p 7, Der p 13 from *Dermatophagoides pteronyssinus* or Der f 5, Der f 7, Der f 13 from *Dermatophagoides farinae.*

A fusion protein according to the invention may also comprise a molecule targeting or activating immune cells. Examples of such molecules include immunoglobulin Fc fragments, the B subunit of cholera toxin or integrins ligands (e.g. Mascarell et al., Vaccine. 2006 Apr 24;24(17):3490-9); Sun et al., Scand J Immunol. 2006 Sep;64(3):251-9).

In other embodiments, the fusion protein could contain, in amino or carboxyterminal position, a tag (for instance a histidine tag), or another moiety which may increase expression, solubility, stability of the molecule, affect the secretion, maturation, post-translational modifications, or facilitate addressing to different subcellular compartments (endoplasmic reticulum, golgi apparatus, endosomes, periplasm...). This additional fragment can be fused so that it can be cleaved off.

### Nucleic acids and methods of expression

The isolated nucleic acids, also named polynucleotides, such as DNA or RNA molecules, that encode the fusion proteins defined above are also part of the invention, while taking into account the degeneracy of the genetic code. They can be obtained by standard techniques well known by the one skilled in the art, such as *in vitro* DNA amplification or polymerisation, *in vitro* gene synthesis, oligonucleotides ligation, or by a combination of these techniques.

Accordingly, the present invention provides a nucleic acid which comprises a sequence coding for a fusion protein according to the invention.

The proteins or nucleic acids of the invention are advantageously in isolated or purified form.

By "purified" and "isolated" it is meant, when referring to a protein or a nucleotide sequence, that the indicated molecule is present in the substantial absence of other biological macromolecules of the same type. The terms "purified" and "isolated" as used herein preferably mean at least 75 % by weight, more preferably at least 85 % by weight, more preferably still at least 95 % by weight, and most preferably at least 98 % by weight, of the protein or nucleotide sequence of interest. An "isolated" or "purified" nucleic acid molecule which encodes a particular polypeptide refers to a nucleic acid molecule which is substantially free of other nucleic acid molecules that do not encode the subject polypeptide. However, the molecule may include some additional bases or moieties which do not deleteriously affect the basic characteristics of the composition.

Briefly, the sequences encoding the polypeptide components of the fusion protein may be assembled separately, and ligated into an appropriate expression vector. The 3' end of the DNA sequence encoding one polypeptide component is ligated, with or without a linker sequence, to the 5' end of a DNA sequence encoding the second polypeptide component so that the reading frames of the sequences are in phase. This permits translation into a single fusion protein that retains the immunogenicity of both component polypeptides.

The DNA fragments encoding the polypeptide components of the fusion protein can also been excised and used as DNA templates for a overlap-PCR with suitable primer pairs. Particularly, the present invention provides a nucleic acid which comprises, consists essentially of, or consists of a fusion of the two sequences SEQ ID NO:1 (mature Der p 1) and SEQ ID NO:2 (mature Der p 2), or of SEQ ID NO:3 (proDer p 1) and SEQ ID NO:2.

As will be understood by those of skill in the art, it may be advantageous in some instances to produce fusion protein-encoding nucleotide molecules possessing codons non-naturally occurring in the components of the fusion. For example, codons preferred by a particular prokaryotic or eukaryotic host can be selected to increase the rate of recombinant protein expression or to produce a recombinant RNA transcript having desirable properties, such as a longer half-life. It also may be advantageous, for example using site-directed mutagenesis, to alter N-glycosylation sites, enzymatic active sites, B or T-cells epitopes, including IgE binding epitopes.

A nucleic acid according to this invention can also include sequences encoding tags, carriers proteins, signal peptides, or non transcribed or translated sequences increasing expression or stability of the molecule.

The invention also provides nucleic acid sequences that are hybridizable to any of the above sequences or their complementary sequences under standard hybridization conditions, preferably conditions of high stringency.

A nucleic acid molecule is "hybridizable" to another nucleic acid molecule, when a single stranded form of the nucleic acid molecule can anneal to the other nucleic acid molecule under the appropriate conditions of temperature and solution ionic strength (see Sambrook et al., 1989). The conditions of temperature and ionic strength determine the "stringency" of the hybridization. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. For hybrids of greater than 100 nucleotides in length, equations for calculating Tm have been derived (see Sambrook et al., supra, 9.50-9.51). A minimum length for a hybridizable nucleic acid is at least about 10 nucleotides ; preferably at least about 15 nucleotides ; and more preferably the length is at least about 20 nucleotides.

In a specific embodiment, the term "standard hybridization conditions" refers to a Tm of 55°C, and utilizes conditions as set forth above. In a preferred embodiment, the Tm is 60°C or even preferred 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

The present invention further relates to cloning and/or expression vectors comprising a nucleic acid sequence of the invention, and to host cells comprising the nucleic acid of the invention or said vector, i.e. a host cell wherein at least one of these nucleic acids or vectors was transferred. The expression vector according to the invention may comprise a functional expression cassette which is also an object of the present invention. An expression cassette comprises a nucleic acid sequence encoding a fusion protein of the invention, which is operably linked to elements necessary to its expression. Said vector advantageously contains a promoter sequence, signals for initiation and termination of translation, as well as appropriate regions for regulation of translation. Its insertion into the host cell may be transient or stable. Said vector may also contain sequences encoding specific signals which trigger the secretion of the translated protein or its targeting to cellular compartments or organelles (e.g; Golgi apparatus, endosomes, periplasm...).

These various control signals are selected according to the host cell and may be inserted into vectors which self-replicate in the host cell, or into vectors which integrate the genome of said host.

Host cells may be prokaryotic or eukaryotic, including but not limited to bacteria, yeasts, plant cells, insect cells, mammalian cells, including cell lines which are commercially available. Preferred examples for expression hosts are *Escherichia coli, Lactobacilli,* probiotic bacteria, *Pichia pastoris, Saccharomyces cerevisiae,* insect cells, plant cells, COS cells and CHO cells.

The transfection of the host cell may be performed using any standard technique, such as chemical transformation, electroporation, phosphate calcium precipitation or lipofection.

Alternatively, the fusion protein according to the invention is expressed *in vitro* with a cell-free transcription and translation system from a DNA or RNA matrix containing required elements for its expression in a cell lysate or reconstituted system (for example, Rapid Translation System®, Roche Diagnostics or Retic Lysate IVT™, Ambion).

The recombinant protein can then be recovered and purified, by means of well-known procedures for purification : it may be purified from lysates or cell extracts, inclusion bodies or from the culture supernatant by methods such as HPLC chromatography, immunoaffinity techniques with specific antibodies, and the like.

### Vaccinal compositions, medicaments and methods for preventing or treating an allergic reaction against mite in an individual

The present invention also relates to the use of a fusion protein as defined above, for the manufacture of a medicament intended for preventing and/or treating a mite allergic reaction.

The present invention also relates to an immune or vaccinal composition comprising a fusion protein according to the invention, in a pharmaceutically acceptable carrier.

The present invention also relates to a method for preventing or treating an allergic reaction comprising administering an individual in need thereof with a prophylactically or therapeutically effective quantity of a fusion protein as defined above.

As used herein, the term "immune composition" denotes a composition which is liable to induce an immune response when administered in an individual.

As intended herein, the term "vaccinal" relates to the capacity of a substance to prevent or to treat a pathological reaction of the immune system.

In the context of the invention, the terms "to treat", "treating" or "treatment", means reversing, alleviating, or inhibiting the course of a pathological reaction of the immune system or one or more symptoms thereof.

In the context of the invention, the terms "to prevent" or "preventing", means the onset of a pathological reaction of the immune system or one or more symptoms thereof.

As used herein, the term "individual" preferably denotes a human, but may more generally a mammal, such as a rodent, a feline, a canine, and a primate.

The suitable immune or vaccinal compositions may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

"Pharmaceutically" or "pharmaceutically acceptable" refers to molecular entities and compositions that do not produce adverse, allergic or other untoward reactions when administered to an animal, or a human, as appropriate.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Its use in the vaccinal compositions, in the medicaments, or for implementing the methods for preventing or treating a pathological reaction of the immune system in an individual according to the invention is contemplated.

In the frame of methods for preventing or treating allergic reactions, the vaccinal compositions or the medicaments, according to the invention, can include any conventional vaccination adjuvant, including heat-labile enterotoxin (LT), cholera-toxin (CT), cholera toxin B subunit (CTB), polymerised liposomes, mutant toxins.

For oromucosal administration, the adjuvants may preferably be a *Bifidobacterium,* a lactic acid bacterium (either in the form of a cell suspension, freeze-dried cells, a lysate, purified sub-components, or purified molecules), or a combination of a corticosteroid with vitamin D3 or any metabolite or analog of the latter.

Advantageously, where mucosal administration is contemplated, the adjuvant may be a synthetic particulate vector that comprises a non-liquid hydrophilic core which comprises a cross-linked polysaccharide. Accordingly, the fusion protein according to the invention may be formulated in a mucoadhesive formulation based on a synthetic particulate vector that comprises (i) a particle comprising a non-liquid hydrophilic core which comprises a cross-linked polysaccharide; and (ii) a fusion protein according to the invention. Such a formulation was found to be particularly efficient in inducing immune tolerance. The particles which can be used are described in the international patent application PCT/IB2007/002379.

Briefly, the cross-linked polysaccharide may be derived from any saccharide monomers, preferably glucose. The polysaccharides preferably have a molecular weight between 2,000 to 100,000 daltons, and most preferably 3,000 to 10,000 daltons. Preferred polysaccharides are starch (glucose alpha 1-4 polymers) and dextran (glucose alpha 1-6 polymers derived from bacteria), or hydrolysates thereof such as dextrins or maltodextrins.

Ionic groups, i.e. anionic (e.g. sulfate or carboxylate) or cationic groups (e.g. quaternary ammonium ions, and primary, secondary, or tertiary amines) are optionally grafted to the core of cross-linked polysaccharide (preferably 0 to 3 milliequivalents, more preferably 0 to 2 milliequivalents, of ionic charge per gram).

Optionally, the cross-linked polysaccharide core is at least partially coated with a layer of amphiphilic compounds and/or a layer of lipidic compounds.

The diameter of the particle may be comprised between 10 nm and 5 µm and preferably between 20 and 200 nm.

For parenteral administration in an aqueous solution, for example, the solution should be suitably buffered if necessary and the liquid diluent first rendered isotonic with sufficient saline or glucose. These particular aqueous solutions are especially suitable for intramuscular and subcutaneous administration. In this connection, sterile aqueous media which can be employed will be known to those of skill in the art in light of the present disclosure.

Preferably, the vaccinal composition, or the medicament is to be administered by the mucosal route, more preferably by the oromucosal route, and most preferably by the sublingual route. As such the vaccinal composition and the medicament are preferably formulated in a way adapted for such administration routes.

Mucosal administration denotes any administration method, wherein the formulation in part or in full comes into contact with a mucosa. Mucosa refers to the epithelial tissue that lines the internal cavities of the body. The mucosal surface may be selected from the group consisting of a nasal, buccal, oral, vaginal, ocular, auditory, pulmonary tract, urethral, digestive tract, and rectal surface.

Oromucosal administration comprises any administration method, wherein the formulation in part or in full comes into contact with the mucosa of the oral cavity and/or the pharynx of the patient. It includes in particular sublingual, perlingual (i.e. through the tongue mucosa) and oral administrations.

In the frame of methods for preventing or treating allergic reactions, the vaccinal compositions or the medicaments, according to the invention, the administration regimen may be maintained for instance for a period of less than 6 weeks to more than 3 years.

Preferably, in the frame of methods for preventing or treating allergic reactions, the range for the vaccinal compositions or the medicaments, according to the invention, may be between 0.001 mg/kg to 5 mg/kg/week. More preferably, the dose range is between 0.01 mg to 1 mg/kg and more preferably 0.03 mg/kg/week to 0.1 mg/kg/week. In preferred embodiments, the dose for the vaccinal compositions or the medicaments according to the invention is 0.056 mg/kg body weight once per week. It is further contemplated that the dose may be between 0.05 mg/kg to 3 mg/kg/week. Some variation in dosage will necessarily occur depending on the condition of the subject being treated. The person responsible for administration will, in any event, determine the appropriate dose for the individual subject.

### Detection / diagnosis applications

The fusion protein according to the invention may further be used to detect antibodies directed against a Group I allergen and/or a Group II allergen from the genus *Dermatophagoides,* in a sample from an individual.

Accordingly, the invention further provides a method for *in vitro* detecting antibodies directed against a Group I allergen and/or Group II allergen, in a biological sample from an individual, which method comprises the steps consisting of:
a) contacting said biological sample from a patient with a fusion protein comprising a Group I allergen and a Group II allergen from the genus *Dermatophagoides,* according to the invention;
b) detecting formation of immune complexes between said fusion protein and antibodies in the biological sample;
whereby, if immune complexes are detected, then the biological sample contains antibodies directed against a Group I allergen and/or a Group II allergen from the genus *Dermatophagoides.*

The individual may be a human or a non-human animal, in particular a non-human mammal, such as a rodent, a feline, a canine, and a primate.

The biological sample may be in particular a biological fluid, such as blood or serum.

Detection of antibodies in the biological sample from an individual may indicate that said individual is sensitised, or allergic, to said Group I allergen and/or a Group II allergen from the genus *Dermatophagoides.*

The antibody may be an IgM, IgE, IgG or IgA antibody.

The skilled person may use any appropriate qualitative or quantitative method known in the art, to detect the antibodies. The assay may be carried out by immobilising the fusion protein on a solid phase, or conversely with the fusion protein is the fluid phase. Typical methods which may be used include ELISA, Western blotting.

Where the concentration of the antibodies is determined, quantitation of the antibody response may be repeated in time, for instance in order to monitor efficacy of a desensitization treatment administered to the individual.

The fusion protein according to the invention may further be used for cellular tests such as a T-cell proliferation test, mediator release test etc. The fusion protein may be exposed to various types of cells in order to elicit measurable responses. Such responses may comprise the release of histamine or other mediators (e.g., leukotriens, serotonine, ECP) in the case of allergic effector cells (e.g:, basophils mast cells, eosinophils). In another type of assay the proliferation or death (e.g., apoptosis) of cells may be measured e.g., by the uptake of .sup.3H Thymidine or any other suitable assay. Such cells may be T cells. Furthermore, fusion proteins may be used to induce the release of cytokines or other immunologically relevant substances (e.g., from T cells) that can be measured. Such cellular tests can be performed for instance on PBMC collected from an individual.

Since fusion proteins can contain epitopes of unrelated allergens they may be used for diagnostic screening tests (in vitro, in vivo as outlined above) in order to detect sensitization or unresponsiveness of an individual against one of the components of the fusion protein. This may allow providing the physician with a diagnostic test which is suited to screen for sensitized patients in a fast way.

Thus the fusion protein according to the invention may also be used for diagnostic purposes, for instance for *in vivo* provocation testing. Such tests may comprise skin testing (e.g., skin prick or intradermal testing), nasal provocation testing, all forms of food challenge testing or bronchial provocation testing.

In particular, the application discloses a method of diagnosis that comprises the steps of (a) injecting by intradermal or subcutaneous route the fusion protein according to the invention; and (b) detecting IgE reactivity, in particular by measuring the diameter of the wheal and flare reaction at the site of injection, wherein an IgE reactivity is indicative of an individual sensitised or allergic to a Group I and/or Group II allergen from the genus *Dermatophagoides.*

The application further discloses a method of detecting an IgE reactivity in an individual which comprises the steps of (a) injecting by intradermal or subcutaneous route the fusion protein according to the invention; and (b) detecting a wheal and flare reaction at the site of injection, wherein a wheal and flare reaction is indicative of an IgE reactivity of the individual to a Group I and/or Group II allergen from the genus *Dermatophagoides.*

The diameter of the wheal and flare reaction at the site of injection may be measured to quantify the IgE reactivity.

The invention also relates to the use of a fusion protein comprising a Group I allergen and a Group II allergen from the genus *Dermatophagoides,* according to the invention, for the manufacture of a diagnostic test. Said diagnostic test further makes part of the invention and is intended to be used for screening of patients sensitized to Group I and/or Group II *Dermatophagoides* allergens.

The invention will be further illustrated in view of the following figures and examples.

### FIGURES

Figure 1: Schematic representation of the Der p 1-Der p 2 fusion proteins

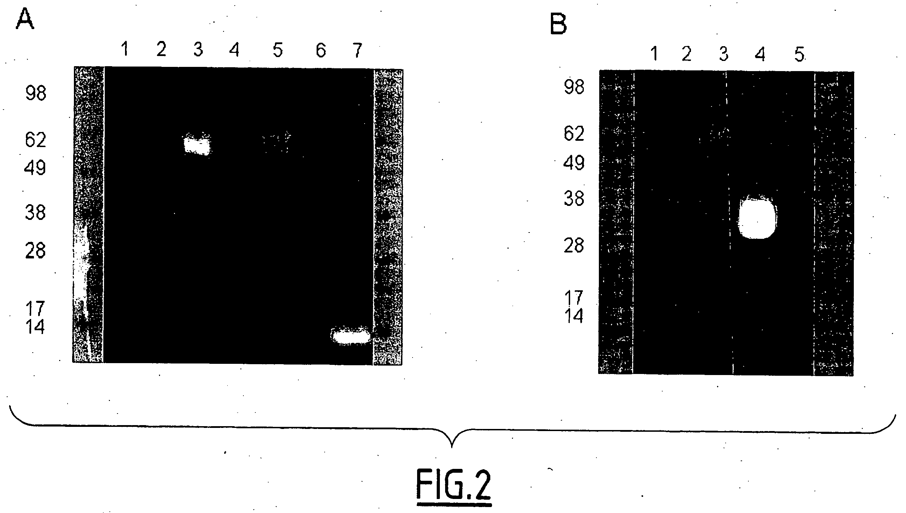

Figure 2: Production of the mite fusion proteins in *Pichia pastoris*
   Western blot analysis of culture supernatants after 15h induction in BMMY medium with specific monoclonal antibodies against the Der p 2 (A) and the Der p 1 (B) moities of the fusion proteins. (A): negative control strain (transformed with pPICZα empty vector; lane 1), F1 (lane 2), F2 (lane 3), F3 (lane 4), F4 (lane 5), recombinant proDer p 1 expressed in tobacco (lane 6, patent WO2004005334), recombinant Der p 2 (lane 7). (B): negative control strain (lane 1), F2 (lane 2), F4 (lane 3), recombinant proDer p 1 (lane 4), recombinant Der p 2 (lane 5). Molecular weights (kDa) are indicated on the left.

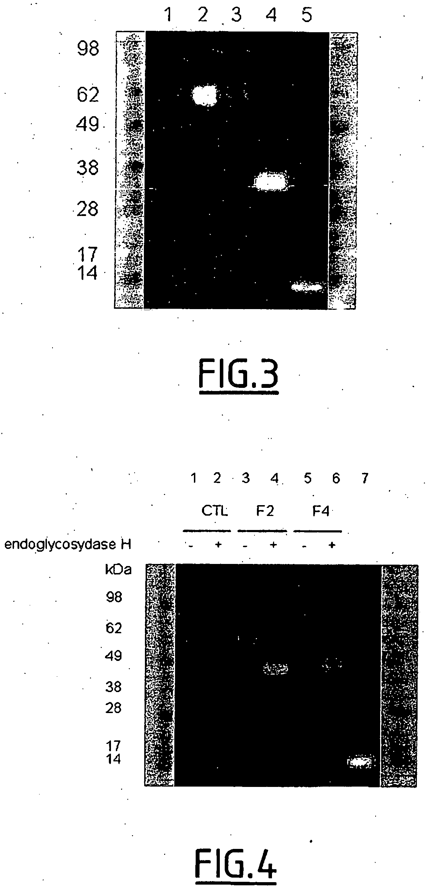

Figure 3: IgE reactivity of the recombinant mite fusion proteins produced in yeast
   Immunoblot analysis of culture supernatants for F2 (lane 2) and F4 (lane 3) with a pool of sera from house dust mite allergic patients. Controls are recombinant Der p 1 (lane 4) and recombinant Der p 2 (lane 5). Molecular weights (kDa) are indicated on the left.
Figure 4: Deglycosylation of mite fusion proteins expressed in yeast
   Buffer exchange of culture supernatant was performed by ultrafiltration. Proteins were treated with endoglycosidase H prior to SDS-PAGE analysis. Non treated (lane 1) and treated (lane 2) negative control supernatant, non treated (lane 3) and treated (lane 4) F2 protein, non treated (lane 5) and treated (lane 6) F4 protein. Lane 7: recombinant Der p 2. Numbers on the left indicate molecular weights (kDa).

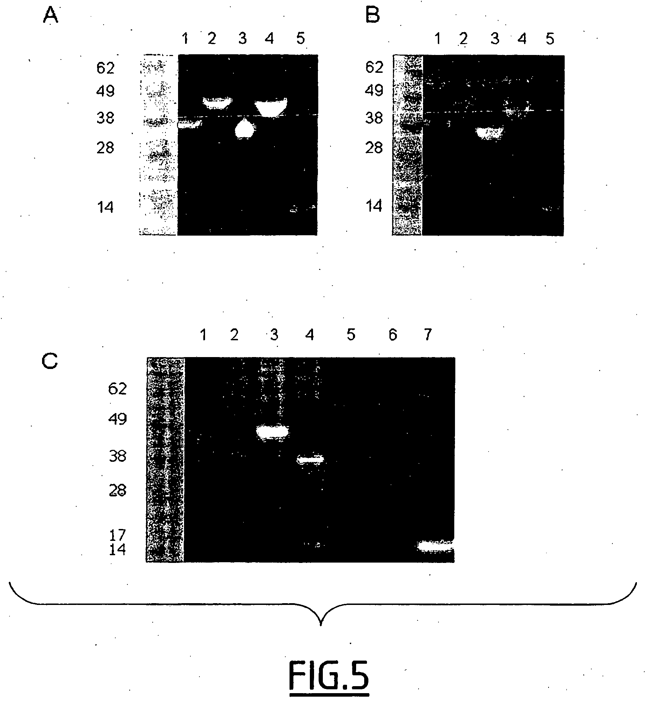

Figure 5: Production of mite fusion proteins in *Escherichia coli*
   Der p 2 (A) and IgE (B) reactivities of *in vitro* expressed mite fusion proteins. F1 (lane 1), F2 (lane 2), F3 (lane 3), F4 (lane 4), recombinant Der p 2 (lane 5, yeast culture supernatant). (C) anti Der p 2 western blot analysis of mite fusion protein expressed *in vivo:* negative control (1), F1 (2), F2 (3), F3 (4), F4 (5), recombinant Der p 1 (6), recombinant Der p 2 (8). Molecular weights (kDa) are indicated on the left sides of the images.

### EXAMPLE

### Materials and methods

### • Construction of mite fusion proteins :

### • For expression in Pichia pastoris

The XhoI-NotI fragments from pPICZα-Der p 1 and pPICZα-Der p 2 (Sanquin, Netherlands) were used to generate the F1 fusion (Der p 2- Der p 1) by an overlap-PCR using the following oligonucleotides: Dp2FW (SEQ ID NO:16 : GGTTGCTCTTCCAACGATCAAGTCGATGTCAAAGAT), Dp2MDp1FW (SEQ ID NO:17 : CATGCTAAAATCCGCGATACTTAACGCCTGCAGTATC), Dp2MDp1 RV (SEQ ID NO:18 : GATACTGCAGGCGTTAGTATCGCGGATTTTAGCATG), Dp1-RV (SEQ ID NO:19 : GCGGCCGCTCAGAGAATGACAACATATGGATA). The PCR fragment was then cloned into pGEM-T vector (Promega). This plasmid subsequently used as a template for PCR with the Dp2_FW_Xhol (SEQ ID NO:20 : GGGCTCGAGAAAAGAGATCAAGTCGATGTCAAAGAT) Dp1_RV_Notl (SEQ ID NO:21 : GGCGGCCGCTCAGAGAATGACAACATATGGATA) primers. The amplified fragment was cloned in frame with the alpha factor propeptide sequence into pPICZα vector (Invitrogen) at the *Xho*I and *Not*I sites. The F2 (Der p 2-proDer p 1), F3 (Der p 1-Der p 2) and F4 (proDer p 1- Der p 2) chimeras were directly cloned into pPICZα plasmid at the *Xho*I and *Not*I sites after an amplification by overlap-PCR using the XhoI-NotI fragments from pPICZα-Der p 1 and pPICZα-Der p 2 (Sanquin, Netherlands) as DNA templates. The primers we used were: Dp2_FW_XhoI, Dp2PDp1 FW (SEQ ID NO:22 : CATGCTAAAATCCGCGATCGTCCATCATCGATCAAA), Dp2PDp1RV (SEQ ID NO:23 : TTTGATCGATGATGGACGATCGCGGATTTTAGCATG) and Dp1_RV_Notl for F2 fusion, Dp1_FW_Xhol (SEQ ID NO:24 : GGGCTCGAGAAAAGAACTAACGCCTGCAGTATC), Dp1Dp2FW (SEQ ID NO:25 : CCATATGTTGTCATTCTCGATCAAGTCGATGTCAAA), Dp1Dp2RV (SEQ ID NO:26 : TTTGACATC-GACTTGATCGAGAATGACAACATATGG), Dp2_RV_NotI (SEQ ID NO:27 : GGCGGCCGCTC-AATCGCGGATTTTAGCATGAGT), for F3 and PDp1_FW_Xhol (SEQ ID NO:28 : GGGCTC-GAGAAAAGACGTCCATCATCGATCAAAACT), Dp1Dp2FW (SEQ ID NO:25 : CCATATGTTGTCAT-TCTCGATCAAGTCGATGTCAAA), Dp1Dp2RV (SEQ ID NO:26 : TTTGACATCGACTTGATCGAGAAT-GACAACATATGG), and Dp2_RV_Notl.

### • For expression in E coli

The different constructs were cloned into pIX2.0 according to the manufacturer instructions (Qiagen). pPICZα derived plasmids described above served as PCR template. The gene-specific primer pairs were His_Dp2_FW (SEQ ID NO:29 : ATCACCATCACCACGGTATGCAAGAT-CAAGTCGATGTCAAA) / Dp1_RV (SEQ ID NO:30 : CTTGGTTAGTTAGTTATTAGAGAATGACAAC-ATATGGATA) for fusions F1 and F2; His_Dp1 (SEQ ID NO:31 : ATCACCATCACCACGGTAT-GCAAACTAACGCCTGCAGTATC) / Dp2_RV (SEQ ID NO:32 : CTTGGTTAGTTAGTTATTAATCG-CGGATTTTAGCATGAGT) for fusion F3 and His_PDp1_FW (SEQ ID NO:33 : ATCACCATCA-CCACGGTATGCAACGTCCATCATCGATCAAA) / Dp2_RV for fusion F4.

### • In vitro expression in E. coli lysate

Proteins were expressed in *E. coli* lysate using the EasyXpress Maxi kit (Qiagen) with 10µg of plasmid DNA template according to the manufacturer instructions and protein contents were analysed by western blot.

### • In vivo expression in E. coli

The fusion proteins were expressed in *E. coli* BL21 (DE3) pLysS strain. Cells were transformed with the different pIX2.0 vectors by the heat shock method according to the provider instructions (Invitrogen). Transformed clones were selected on LB plates containing Carbenicillin (50µg/ml) and Chloramphenicol (20µg/ml). The construct integrity was checked by PCR.

Single colonies were inoculated in LB Cb (50µg/ml) Cam (20µg/ml) medium and grown overnight at 37°C. Overnight cultures were diluted 1:100 in LB Cb (50µg/ml) and grown until the OD₆₀₀ₙₘ was between 0.4 and 0.8. Target protein expression was then induced with 1 mM IPTG (Sigma Aldrich). Cell pellets were collected by centrifugation and stored at -20°C for further analysis. Lysis was performed according to the following procedure. Cell pellets were first frozen in liquid nitrogen and thawed at 37°C five times, resuspended in lysis buffer (50mM Tris pH8 50mM NaCl 1mM EDTA) and incubated on a tube rotator for 30 minutes at 4°C. Samples were then sonicated (10 times 10 seconds, 50W, on ice) and clarified by centrifugation. Lysis supernatants were submitted to western blot analysis.

### • Expression in yeast

The fusion proteins were expressed in methanotrophic *P. pastoris* GS115 or X33 strains. Transformation was performed according to the manufacturer instructions (Invitrogen). Briefly, pPICZα plasmids containing the different constructs were digested with *SacI* and digested plasmids were used to transform yeast cells by electroporation. Positive clones were selected on YPD zeocine (100µg/ml) plates and integration of the constructs was checked by PCR.

Selected clones were inoculated in buffered glycerol complex medium (BMGY pH6) and grown overnight at 30°C to an OD₆₀₀ₙₘ of 2-6. Cells were then diluted in buffered methanol complex medium (BMMY pH6) at an OD₆₀₀ₙₘ of 1 and growth was continued for 15 hours at 30°C. Culture supernatant was harvested by centrifugation and stored at -20°C until use.

### • SDS-PAGE and western blot analysis

Proteins were separated by SDS-PAGE using Nupage 4-12% Bis-Tris acrylamide gels with MES buffer under non-reducing conditions and then transferred onto nitrocellulose membranes as described by the manufacturer (Invitrogen). The molecular size marker is SeeBlue Plus2 (Invitrogen). Unreacted sites were blocked by incubating membranes in TBS buffer containing 1% non fat milk (BioRad). Reactivities of the Der p 2 and Der p 1 moities were then analysed with an anti Dpx antibody (0.2µg/ml; Indoor Biotechnologies, USA), an in-house mouse monoclonal anti Der p 1 antibody (0.25 µg/ml) or serum pool from allergic patients (1:30). Incubations were done in TBS buffer supplemented with 0.2% non fat milk and 0.1% Tween 20. The secondary antibodies were an HRP conjugated sheep anti mouse IgG antibody (Sigma), or a rabbit anti human IgE antibody (Dakota) followed by an HRP conjugated goat anti rabbit IgG antibody (Calbiochem). For detection, we used either the West Pico or West Femto chemiluminescent substrates (Molecular Probes).

### • Endoglycosidase H treatment

Proteins were placed in a 50mM Sodium Acetate pH5.2 buffer by buffer exchange with Vivaspin 500 filters (PES MWCO 10 kDa, Sartorius). 0.04% SDS was subsequently added. 50 µl samples were incubated with or without 5 mU of endoglycosidase H (Roche Diagnostics) at 37°C for 4 hours. Proteins were then analysed by SDS-PAGE.

### Abbreviations:

Cb: carbenicillin, Cam: chloramphenicol

### Results

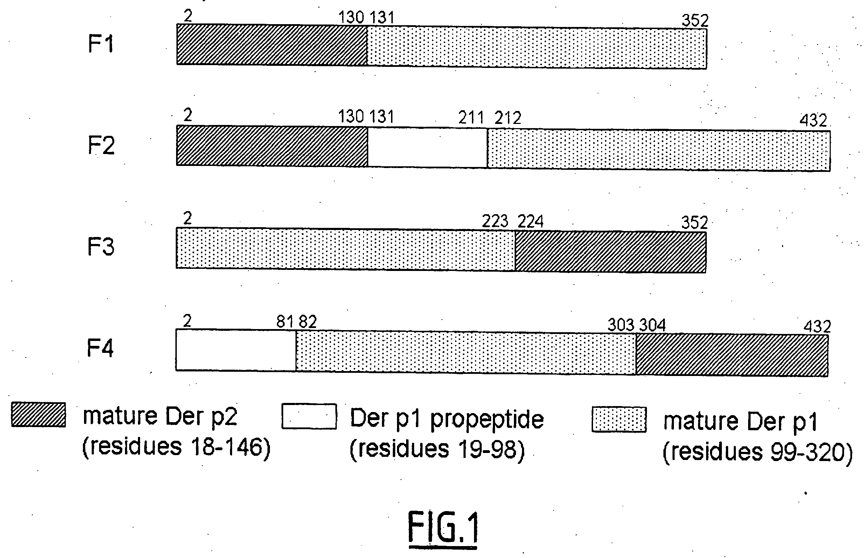

N-terminal and C-terminal fusions of Der p 2 with either mature Der p 1 or proDer p 1 were constructed. Boxes in Figure 1 represent the different domains: Der p 2 (hatched), Der p 1 propeptide (white), mature Der p 1 (dotted). The numbers indicate the positions of the boundaries of these different moieties in the fusion proteins.

The four F1, F2, F3 and F4 fusion proteins have been expressed in *E. coli.* Those proteins are recognized by an anti-Der p 2 antibody and IgE from house dust mite allergic patients.

F2 and F4 fusion proteins have been successfully expressed in *P. pastoris.* These proteins are recognised by both anti Der p 1 and Der p 2 antibodies and by patients IgE. When expressed in yeast, F2 and F4 fusion proteins are hyperglycosylated. Their carbohydrates have been removed by enzymatic treatment with endoglycosidase H.

## Claims

1. A fusion protein comprising a Group I allergen and a Group II allergen from the genus *Dermatophagoides,*
wherein said Group I allergen from the genus *Dermatophagoides* is selected from the group consisting of Der p 1, Der f 1, a proform of Der p 1, a proform of Der f 1, and a sequence at least 80% identical to SEQ ID NO:5 or SEQ ID NO:7 which substantially retains the immunogenicity of the polypeptide of sequence SEQ ID NO:5 or SEQ ID NO:7, respectively; and
wherein said Group II allergen from the genus *Dermatophagoides* is selected from the group consisting of Der p 2, Der f 2, a proform of Der p 2, a proform of Derf 2, and a sequence at least 80% identical to SEQ ID NO:4 which substantially retains the immunogenicity of the polypeptide of sequence SEQ ID NO:4.

2. A fusion protein according to claim 1, wherein the Group II allergen is fused at the N- or C-terminal end of the Group I allergen.

3. A fusion protein according to claim 1 or 2, wherein said Group I allergen is Der p 1.

4. A fusion protein according to claim 1 or 2, wherein said Group I allergen is Der p 1 or proDer p 1, and said Group II allergen is Der p 2.

5. A fusion protein according to claim 1 or 2, wherein said Group I allergen is Der f 1 or proDer f 1 and said Group II allergen is Der f 2.

6. A fusion protein according to claim 4, comprising a sequence selected from the group consisting of SEQ ID NO:4, SEQ ID NO:5, SEQ ID NO:6 and SEQ ID NO:7.

7. A fusion protein according to any of claims 1 to 6, comprising at least one additional allergen from the genus *Dermatophagoides.*

8. A fusion protein according to any of claims 1 to 7, comprising at least one addressing molecule targeting immune cells.

9. A nucleic acid comprising a sequence coding for a fusion protein according to any of claims 1 to 8.

10. A nucleic acid comprising a sequence consisting of a fusion of SEQ ID NO:1 and SEQ ID NO:2, or SEQ ID NO:3 and SEQ ID NO:2.

11. A nucleic acid comprising a sequence selected from the group consisting of SEQ ID NO:8, SEQ ID NO:9, SEQ ID NO:10 and SEQ ID NO:11.

12. An expression cassette comprising a nucleic acid as defined in any of claims 9 to 11 placed under the control of the elements necessary for its expression in a prokaryotic or eukaryotic cell or in an *in vitro* cell-free system.

13. An expression cassette according to claim 12, **characterized in that** the prokaryotic or eukaryotic cell is selected from cells of *Escherichia coli, Pichia pastoris, Saccharomyces cerevisiae,* COS and CHO cells.

14. A cloning and/or expression vector containing an expression cassette according to any of claims 12 or 13.

15. A prokaryotic or eukaryotic cell containing an expression cassette according to any of claims 12 or 13 or a vector according to claim 14.

16. A prokaryotic or eukaryotic cell according to claim 15 selected from the group consisting of a cell from *Escherichia coli, Pichia pastoris* or *Saccharomyces cerevisiae,* a COS cell, a CHO cell.

17. An immune or vaccinal composition comprising a fusion protein as defined in any of claims 1 to 8, in a pharmaceutically acceptable carrier.

18. An immune or vaccinal composition according to claim 17, further comprising a pharmaceutically acceptable adjuvant.

19. The immune or vaccinal composition according to claim 18, wherein the pharmaceutically acceptable adjuvant is a synthetic particulate vector that comprises a non-liquid hydrophilic core which comprises a cross-linked polysaccharide.

20. A fusion protein as defined in any of claims 1 to 8, for use in the prevention and/or treatment of a mite allergic reaction.

21. A method for *in vitro* detecting antibodies directed against a Group I allergen and/or Group II allergen, in a biological sample from an individual, which method comprises the steps consisting of:
a) contacting said biological sample from a patient with a fusion protein comprising a Group I allergen and a Group II allergen from the genus *Dermatophagoides,*
wherein said Group I allergen from the genus *Dermatophagoides* is selected from the group consisting of Der p 1, Der f 1, a proform of Der p 1, a proform of Der f 1, and a sequence at least 80% identical to SEQ ID NO:5 or SEQ ID NO:7 which substantially retains the immunogenicity of the polypeptide of sequence SEQ ID NO:5 or SEQ ID NO:7, respectively; and
wherein said Group II allergen from the genus *Dermatophagoides* is selected from the group consisting of Der p 2, Der f 2, a proform of Der p 2, a proform of Der f 2, and a sequence at least 80% identical to SEQ ID NO:4 which substantially retains the immunogenicity of the polypeptide of sequence SEQ ID NO:4;
b) detecting formation of immune complexes between said fusion protein and antibodies in the biological sample;
whereby, if immune complexes are detected, then the biological sample contains antibodies directed against a Group I allergen and/or a Group II allergen from the genus *Dermatophagoides.*

22. A diagnostic test for screening patients sensitized to Group I and/or Group II *Dermatophagoides* allergens, which comprises a fusion protein according to any one of claims 1 to 8.

## Patentansprüche

1. Fusionsprotein, umfassend ein Allergen der Gruppe I und ein Allergen der Gruppe II der Gattung *Dermatophagoides,*
- wobei das Allergen der Gruppe I der Gattung *Dermatophagoides* aus der nachfolgenden Gruppe gewählt ist: Der p 1, Der f 1, eine Proform von Der p 1, eine Proform von Der f 1 und eine Sequenz, die mindestens 80 % Identität mit SEQ ID NR:5 oder SEQ ID NR:7 aufweist und die Immunogenität des Polypeptids jeweils der Sequenz SEQ ID NR:5 oder SEQ ID NR:7 im Wesentlichen beibehält, und
- wobei das Allergen der Gruppe II der Gattung *Dermatophagoides* aus der nachfolgenden Gruppe gewählt ist: Der p 2, Der f 2, eine Proform von Der p 2, eine Proform von Der f 2 und eine Sequenz, die mindestens 80 % Identität mit SEQ ID NR:4 aufweist und die Immunogenität des Polypeptids der Sequenz SEQ ID NR:4 im Wesentlichen beibehält.

2. Fusionsprotein nach Anspruch 1, wobei das Allergen der Gruppe II am N- oder C-terminalen Ende des Allergens der Gruppe I fusioniert ist.

3. Fusionsprotein nach Anspruch 1 oder 2, wobei das Allergen der Gruppe I Der p 1 ist.

4. Fusionsprotein nach Anspruch 1 oder 2, wobei das Allergen der Gruppe I Der p 1 oder proDer p 1 und das Allergen der Gruppe II Der p 2 ist.

5. Fusionsprotein nach Anspruch 1 oder 2, wobei das Allergen der Gruppe I Der f 1 oder proDer f 1 und das Allergen der Gruppe II Der f 2 ist.

6. Fusionsprotein nach Anspruch 4, umfassend eine Sequenz aus der nachfolgenden Gruppe: SEQ ID NR:4, SEQ ID NR:5, SEQ ID NR:6 und SEQ ID NR:7.

7. Fusionsprotein nach einem der Ansprüche 1 - 6, umfassend mindestens ein zusätzliches Allergen der Gattung *Dermatophagoides.*

8. Fusionsprotein nach einem der Ansprüche 1 - 7, umfassend mindestens ein Adressierungsmolekül, das auf Immunzellen zielt.

9. Nukleinsäure, umfassend eine Sequenz, die für ein Fusionsprotein nach einem der Ansprüche 1 - 8 codiert.

10. Nukleinsäure, umfassend eine Sequenz, die aus einer Fusion von SEQ ID NR:1 und SEQ ID NR:2 oder SEQ ID NR:3 und SEQ ID NR:2 besteht.

11. Nukleinsäure, umfassend eine Sequenz aus der nachfolgenden Gruppe: SEQ ID NR:8, SEQ ID NR:9, SEQ ID NR:10 und SEQ ID NR:11.

12. Expressionskassette, umfassend eine Nukleinsäure nach einem der Ansprüche 9 - 11, die der Kontrolle der zu ihrer Expression erforderlichen Elemente in einer prokaryotischen oder eukaryotischen Zelle oder einem zellfreien *in vitro-*System unterstellt wird.

13. Expressionskassette nach Anspruch 12, **dadurch gekennzeichnet, dass** die prokaryotische oder eukaryotische Zelle aus der nachfolgenden Gruppe gewählt ist: Zellen von *Escherichia coli, Pichia pastotis, Saccharomyces cerevisiae,* COS- und CHO-Zellen.

14. Klonungs- und/oder Expressionsvektor, der eine Expressionskassette nach einem der Ansprüche 12 oder 13 enthält.

15. Prokaryotische oder eukaryotische Zelle, die eine Expressionskassette nach einem der Ansprüche 12 oder 13 oder einen Vektor nach Anspruch 14 enthält.

16. Prokaryotische oder eukaryotische Zelle nach Anspruch 15, die aus der nachfolgenden Gruppe gewählt ist: eine Zelle aus *Escherichia coli, Pichia pastotis oder Saccharomyces cerevisiae,* eine COS-Zelle, eine CHO-Zelle.

17. Immun- oder Impfstoffzusammensetzung, umfassend ein Fusionsprotein nach einem der Ansprüche 1 - 8 in einem pharmazeutisch annehmbaren Träger.

18. Immun- oder Impfstoffzusammensetzung nach Anspruch 17, ferner umfassend einen pharmazeutisch annehmbaren Hilfsstoff.

19. Immun- oder Impfstoffzusammensetzung nach Anspruch 18, wobei der pharmazeutisch annehmbare Hilfstoff ein synthetischer Partikelvektor ist, der einen nicht flüssigen hydrophilen Kern umfasst, der ein vernetztes Polysaccharid umfasst.

20. Fusionsprotein nach einem der Ansprüche 1 - 8 zum Einsatz bei der Vorbeugung und/oder Behandlung einer allergischen Reaktion auf Milben.

21. Verfahren zur *in vitro*-Erkennung von Antikörpern, die gegen ein Allergen der Gruppe I und/oder ein Allergen der Gruppe II gerichtet sind, in einer biologischen Probe aus einer Person, umfassend die nachfolgenden Schritte:
a) Kontaktieren der biologischen Probe aus einem Patienten mit einem Fusionsprotein, das ein Allergen der Gruppe I und ein Allergen der Gruppe II der Gattung *Dermatophagoides* umfasst,
- wobei das Allergen der Gruppe I der Gattung *Dermatophagoides* aus der nachfolgenden Gruppe gewählt ist: Der p 1, Der f 1, eine Proform von Der p 1, eine Proform von Der f 1 und eine Sequenz, die mindestens 80 % Identität mit SEQ ID NR:5 oder SEQ ID NR:7 aufweist und die Immunogenität des Polypeptids jeweils der Sequenz SEQ ID NR:5 oder SEQ ID NR:7 im Wesentlichen beibehält, und
- wobei das Allergen der Gruppe II der Gattung *Dermatophagoides* aus der nachfolgenden Gruppe gewählt ist: Der p 2, Der f 2, eine Proform von Der p 2, eine Proform von Der f 2 und eine Sequenz, die mindestens 80 % Identität mit SEQ ID NR:4 aufweist und die Immunogenität des Polypeptids der Sequenz SEQ ID NR:4 im Wesentlichen beibehält;
b) Erkennen der Bildung von Immunkomplexen zwischen dem Fusionsprotein und Antikörpern in der biologischen Probe,
wobei, sofern Immunkomplexe erkannt werden, die biologische Probe dann Antikörper enthält, die gegen ein Allergen der Gruppe I und/oder ein Allergen der Gruppe II der Gattung *Dermatophagoides* gerichtet sind.

22. Diagnostische Untersuchung zur Untersuchung von Patienten, die gegenüber *Dermatophagoides*-Allergenen der Gruppe I und/oder der Gruppe II sensibilisiert sind, umfassend ein Fusionsprotein nach einem der Ansprüche 1 - 8.

## Revendications

1. Protéine de fusion comprenant un allergène du groupe I et un allergène du groupe II issu du genre *Dermatophagoides,*
- dans laquelle ledit allergène du groupe I issu du genre *Dermatophagoides* est sélectionné dans le groupe consistant en Der p 1, Der f 1, une proforme de Der p 1, une proforme de Der f 1, et une séquence au moins 80 % identique à SEQ ID NO:5 ou SEQ ID NO:7 qui conserve essentiellement l'immunogénicité du polypeptide de séquence SEQ ID NO: 5 ou SEQ ID NO: 7, respectivement ; et
- dans laquelle ledit allergène du groupe II issu du genre *Dermatophagoides* est sélectionné dans le groupe consistant en Der p 2, Der f 2, une proforme de Der p 2, une proforme de Der f 2, et une séquence au moins 80 % identique à SEQ ID NO:4 qui conserve essentiellement l'immunogénicité du polypeptide de séquence SEQ ID NO:4.

2. Protéine de fusion selon la revendication 1, dans laquelle l'allergène du groupe II est fusionné à l'extrémité N- ou C-terminale de l'allergène du groupe I.

3. Protéine de fusion selon la revendication 1 ou 2, dans laquelle ledit allergène du groupe I est Der p 1.

4. Protéine de fusion selon la revendication 1 ou 2, dans laquelle ledit allergène du groupe I est Der p 1 ou proDer p 1, et ledit allergène du groupe II est Der p 2.

5. Protéine de fusion selon la revendication 1 ou 2, dans laquelle ledit allergène du groupe I est Der f 1 ou proDer f 1 et ledit allergène du groupe II est Der f 2.

6. Protéine de fusion selon la revendication 4, comprenant une séquence sélectionnée dans le groupe consistant en SEQ ID NO: 4, SEQ ID NO: 5, SEQ ID NO: 6 et SEQ ID NO:7.

7. Protéine de fusion selon l'une quelconque des revendications 1 à 6, comprenant au moins un allergène supplémentaire issu du genre *Dermatophagoides.*

8. Protéine de fusion selon l'une quelconque des revendications 1 à 7, comprenant au moins une molécule d'adressage ciblant des cellules immunitaires.

9. Acide nucléique comprenant une séquence codant pour une protéine de fusion selon l'une quelconque des revendications 1 à 8.

10. Acide nucléique comprenant une séquence consistant en une fusion de SEQ ID NO: 1 et SEQ ID NO: 2, ou SEQ ID NO: 3 et SEQ ID NO: 2.

11. Acide nucléique comprenant une séquence sélectionnée dans le groupe consistant en SEQ ID NO: 8, SEQ ID NO: 9, SEQ ID NO: 10 et SEQ ID NO: 11.

12. Cassette d'expression comprenant un acide nucléique tel que défini dans l'une quelconque des revendications 9 à 11 placé sous le contrôle des éléments nécessaires pour son expression dans une cellule procaryote ou eucaryote ou dans un système acellulaire *in vitro.*

13. Cassette d'expression selon la revendication 12, **caractérisée en ce que** la cellule procaryote ou eucaryote est sélectionnée parmi des cellules d'*Escherichia coli,* de *Pichia pastoris,* de *Saccharomyces cerevisiae,* des cellules COS et CHO.

14. Vecteur de clonage et/ou d'expression contenant une cassette d'expression selon l'une quelconque des revendications 12 ou 13.

15. Cellule procaryote ou eucaryote contenant une cassette d'expression selon l'une quelconque des revendications 12 ou 13 ou un vecteur selon la revendication 14.

16. Cellule procaryote ou eucaryote selon la revendication 15 sélectionnée dans le groupe consistant en une cellule *d'Escherichia coli,* de *Pichia pastoris* ou de *Saccharomyces cerevisiae,* une cellule COS, une cellule CHO.

17. Composition immunitaire ou vaccinale comprenant une protéine de fusion telle que définie dans l'une quelconque des revendications 1 à 8, dans un véhicule pharmaceutiquement acceptable.

18. Composition immunitaire ou vaccinale selon la revendication 17, comprenant en outre un adjuvant pharmaceutiquement acceptable.

19. Composition immunitaire ou vaccinale selon la revendication 18, dans laquelle l'adjuvant pharmaceutiquement acceptable est un vecteur particulaire synthétique qui comprend un noyau hydrophile non liquide qui comprend un polysaccharide réticulé.

20. Protéine de fusion telle que définie dans l'une quelconque des revendications 1 à 8, destinée à être utilisée dans la prévention et/ou le traitement d'une réaction allergique aux acariens.

21. Procédé de détection *in vitro* d'anticorps dirigés contre un allergène du groupe I et/ou un allergène du groupe II, dans un échantillon biologique d'un individu, le procédé comprenant les étapes consistant à :
a) mettre en contact ledit échantillon biologique d'un patient avec une protéine de fusion comprenant un allergène du groupe I et un allergène du groupe II issu du genre *Dermatophagoides,*
- dans laquelle ledit allergène du groupe I issu du genre *Dermatophagoides* est sélectionné dans le groupe consistant en Der p 1, Der f 1, une proforme de Der p 1, une proforme de Der f 1, et une séquence au moins 80 % identique à SEQ ID NO:5 ou SEQ ID NO:7 qui conserve essentiellement l'immunogénicité du polypeptide de séquence SEQ ID NO: 5 ou SEQ ID NO: 7, respectivement ; et
- dans laquelle ledit allergène du groupe II issu du genre *Dermatophagoides* est sélectionné dans le groupe consistant en Der p 2, Der f 2, une proforme de Der p 2, une proforme de Der f 2, et une séquence au moins 80 % identique à SEQ ID NO:4 qui conserve essentiellement l'immunogénicité du polypeptide de séquence SEQ ID NO:4;
b) détecter la formation de complexes immuns entre ladite protéine de fusion et des anticorps dans l'échantillon biologique ;
moyennant quoi, si des complexes immuns sont détectés, alors l'échantillon biologique contient des anticorps dirigés contre un allergène du groupe I et/ou un allergène du groupe II issu du genre *Dermatophagoides.*

22. Test diagnostique pour dépister des patients sensibilisés à des allergènes du groupe I et/ou du groupe II de *Dermatophagoides,* qui comprend une protéine de fusion selon l'une quelconque des revendications 1 à 8.


### SEQUENCE LISTING

<110> STALLERGENES SA
<120> Mite Fusion Proteins
<130> BET07P1253
<160> 33
<170> Patent In version 3.2
<210> 1
   <211> 666
   <212> DNA
   <213> Dermatophagoides pteronyssinus
<400> 1
   actaacgcct gcagtatcaa tggaaatgct ccagctgaaa tcgatttgcg acaaatgcga      60
   actgtcactc ccattcgtat gcaaggaggc tgtggttcat gttgggcttt ctctggtgtt     120
   gccgcaactg aatcagctta tttggcttac cgtaatcaat cattggatct tgctgaacaa     180
   gaattagtcg attgtgcttc ccaacacggt tgtcatggtg ataccattcc acgtggtatt     240
   gaatacatcc aacataatgg tgtcgtccaa gaaagctact atcgatacgt tgcacgagaa     300
   caatcatgcc gacgaccaaa tgcacaacgt ttcggtatct caaactattg ccaaatttac     360
   ccaccaaatg caaacaaaat tcgtgaagct ttggctcaaa cccacagcgc tattgccgtc     420
   attattggca tcaaagattt agacgcattc cgtcattatg atggccgaac aatcattcaa     480
   cgcgataatg gttaccaacc aaactatcac gctgtcaaca ttgttggtta cagtaacgca     540
   caaggtgtcg attattggat cgtacgaaac agttgggata ccaattgggg tgataatggt     600
   tacggttatt ttgctgccaa catcgatttg atgatgattg aagaatatcc atatgttgtc     660
   attctc                                                                666
<210> 2
   <211> 387
   <212> DNA
   <213> Dermatophagoides pteronyssinus
<400> 2
   gatcaagtcg atgtcaaaga ttgtgccaat catgaaatca aaaaagtttt ggtaccagga      60
   tgccatggtt cagaaccatg tatcattcat cgtggtaaac cattccaatt ggaagccgtt     120
   ttcgaagcca accaaaacac aaaaacggct aaaattgaaa tcaaagcctc aatcgatggt     180
   ttagaagttg atgttcccgg tatcgatcca aatgcatgcc attacatgaa atgcccattg     240
   gttaaaggac aacaatatga tattaaatat acatggaatg ttccgaaaat tgcaccaaaa     300
   tctgaaaatg ttgtcgtcac tgttaaagtt atgggtgatg atggtgtttt ggcctgtgct     360
   attgctactc atgctaaaat ccgcgat                                         387
<210> 3
   <211> 906
   <212> DNA
   <213> Dermatophagoides pteronyssinus
<400> 3
   cgtccatcat cgatcaaaac ttttgaagaa tacaaaaaag ccttcaacaa aagttatgct     60
   accttcgaag atgaagaagc tgcccgtaaa aactttttgg aatcagtaaa atatgttcaa    120
   tcaaatggag gtgccatcaa ccatttgtcc gatttgtcgt tggatgaatt caaaaaccga    180
   tttttgatga gtgcagaagc ttttgaacac ctcaaaactc aattcgattt gaatgctgaa    240
   actaacgcct gcagtatcaa tggaaatgct ccagctgaaa tcgatttgcg acaaatgcga    300
   actgtcactc ccattcgtat gcaaggaggc tgtggttcat gttgggcttt ctctggtgtt    360
   gccgcaactg aatcagctta tttggcttac cgtaatcaat cattggatct tgctgaacaa    420
   gaattagtcg attgtgcttc ccaacacggt tgtcatggtg ataccattcc acgtggtatt    480
   gaatacatcc aacataatgg tgtcgtccaa gaaagctact atcgatacgt tgcacgagaa    540
   caatcatgcc gacgaccaaa tgcacaacgt ttcggtatct caaactattg ccaaatttac    600
   ccaccaaatg caaacaaaat tcgtgaagct ttggctcaaa cccacagcgc tattgccgtc    660
   attattggca tcaaagattt agacgcattc cgtcattatg atggccgaac aatcattcaa    720
   cgcgataatg gttaccaacc aaactatcac gctgtcaaca ttgttggtta cagtaacgca    780
   caaggtgtcg attattggat cgtacgaaac agttgggata ccaattgggg tgataatggt    840
   tacggttatt ttgctgccaa catcgatttg atgatgattg aagaatatcc atatgttgtc    900
   attctc                                                               906
<210> 4
   <211> 129
   <212> PRT
   <213> Dermatophagoides pteronyssinus
<400> 4
   Asp Gln Val Asp Val Lys Asp Cys Ala Asn His Glu Ile Lys Lys Val
   1               5                   10                  15
   Leu Val Pro Gly Cys His Gly Ser Glu Pro Cys Ile Ile His Arg Gly
               20                  25                  30
   Lys Pro Phe Gln Leu Glu Ala Val Phe Glu Ala Asn Gln Asn Thr Lys
           35                  40                  45
   Thr Ala Lys Ile Glu Ile Lys Ala Ser Ile Asp Gly Leu Glu Val Asp
   50 55 60
   Val Pro Gly Ile Asp Pro Asn Ala Cys His Tyr Met Lys Cys Pro Leu
   65 70 75 80
   Val Lys Gly Gln Gln Tyr Asp Ile Lys Tyr Thr Trp Asn Val Pro Lys
   85 90 95
   Ile Ala Pro Lys Ser Glu Asn Val Val Val Thr Val Lys Val Met Gly
   100 105 110
   Asp Asp Gly Val Leu Ala Cys Ala Ile Ala Thr His Ala Lys Ile Arg
   115 120 125
   Asp
<210> 5
   <211> 222
   <212> PRT
   <213> Dermatophagoides pteronyssinus
<400> 5
   Thr Asn Ala Cys Ser Ile Asn Gly Asn Ala Pro Ala Glu Ile Asp Leu
   1 5 10 15
   Arg Gln Met Arg Thr Val Thr Pro Ile Arg Met Gln Gly Gly Cys Gly
   20 25 30
   Ser Cys Trp Ala Phe Ser Gly Val Ala Ala Thr Glu Ser Ala Tyr Leu
   35 40 45
   Ala Tyr Arg Asn Gln Ser Leu Asp Leu Ala Glu Gln Glu Leu Val Asp
   50 55 60
   Cys Ala Ser Gln His Gly Cys His Gly Asp Thr Ile Pro Arg Gly Ile
   65 70 75 80
   Glu Tyr Ile Gln His Asn Gly Val Val Gln Glu Ser Tyr Tyr Arg Tyr
   85 90 95
   Val Ala Arg Glu Gln Ser Cys Arg Arg Pro Asn Ala Gln Arg Phe Gly
   100 105 110
   Ile Ser Asn Tyr Cys Gln Ile Tyr Pro Pro Asn Ala Asn Lys Ile Arg
   115 120 125
   Glu Ala Leu Ala Gln Thr His Ser Ala Ile Ala Val Ile Ile Gly Ile
   130 135 140
   Lys Asp Leu Asp Ala Phe Arg His Tyr Asp Gly Arg Thr Ile Ile Gln
   145 150 155 160
   Arg Asp Asn Gly Tyr Gln Pro Asn Tyr His Ala Val Asn Ile Val Gly
   165 170 175
   Tyr Ser Asn Ala Gln Gly Val Asp Tyr Trp Ile Val Arg Asn Ser Trp
   180 185 190
   Asp Thr Asn Trp Gly Asp Asn Gly Tyr Gly Tyr Phe Ala Ala Asn Ile
   195 200 205
   Asp Leu Met Met Ile Glu Glu Tyr Pro Tyr Val Val Ile Leu
   210 215 220
<210> 6
   <211> 320
   <212> PRT
   <213> Dermatophagoides pteronyssinus
<400> 6
   Met Lys Ile Val Leu Ala Ile Ala Ser Leu Leu Ala Leu Ser Ala Val
   1 5 10 15
   Tyr Ala Arg Pro Ser Ser Ile Lys Thr Phe Glu Glu Tyr Lys Lys Ala
   20 25 30
   Phe Asn Lys Ser Tyr Ala Thr Phe Glu Asp Glu Glu Ala Ala Arg Lys
   35 40 45
   Asn Phe Leu Glu Ser Val Lys Tyr Val Gln Ser Asn Gly Gly Ala Ile
   50 55 60
   Asn His Leu Ser Asp Leu Ser Leu Asp Glu Phe Lys Asn Arg Phe Leu
   65 70 75 80
   Met Ser Ala Glu Ala Phe Glu His Leu Lys Thr Gln Phe Asp Leu Asn
   85 90 95
   Ala Glu Thr Asn Ala Cys Ser Ile Asn Gly Asn Ala Pro Ala Glu Ile
   100 105 110
   Asp Leu Arg Gln Met Arg Thr Val Thr Pro Ile Arg Met Gln Gly Gly
   115 120 125
   Cys Gly Ser Cys Trp Ala Phe Ser Gly Val Ala Ala Thr Glu Ser Ala
   130 135 140
   Tyr Leu Ala Tyr Arg Asn Gln Ser Leu Asp Leu Ala Glu Gln Glu Leu
   145 150 155 160
   Val Asp Cys Ala Ser Gln His Gly Cys His Gly Asp Thr Ile Pro Arg
   165 170 175
   Gly Ile Glu Tyr Ile Gln His Asn Gly Val Val Gln Glu Ser Tyr Tyr
   180 185 190
   Arg Tyr Val Ala Arg Glu Gln Ser Cys Arg Arg Pro Asn Ala Gln Arg
   195 200 205
   Phe Gly Ile Ser Asn Tyr Cys Gln Ile Tyr Pro Pro Asn Ala Asn Lys
   210 215 220
   Ile Arg Glu Ala Leu Ala Gln Thr His Ser Ala Ile Ala Val Ile Ile
   225 230 235 240
   Gly Ile Lys Asp Leu Asp Ala Phe Arg His Tyr Asp Gly Arg Thr Ile
   245 250 255
   Ile Gln Arg Asp Asn Gly Tyr Gln Pro Asn Tyr His Ala Val Asn Ile
   260 265 270
   Val Gly Tyr Ser Asn Ala Gln Gly Val Asp Tyr Trp Ile Val Arg Asn
   275 280 285
   Ser Trp Asp Thr Asn Trp Gly Asp Asn Gly Tyr Gly Tyr Phe Ala Ala
   290 295 300
   Asn Ile Asp Leu Met Met Ile Glu Glu Tyr Pro Tyr Val Val Ile Leu
   305 310 315 320
<210> 7
   <211> 302
   <212> PRT
   <213> Dermatophagoides pteronyssinus
<400> 7
   Arg Pro Ser Ser Ile Lys Thr Phe Glu Glu Tyr Lys Lys Ala Phe Asn
   1               5                   10                  15
   Lys Ser Tyr Ala Thr Phe Glu Asp Glu Glu Ala Ala Arg Lys Asn Phe
               20                  25                  30
   Leu Glu Ser Val Lys Tyr Val Gln Ser Asn Gly Gly Ala Ile Asn His
           35                  40                  45
   Leu Ser Asp Leu Ser Leu Asp Glu Phe Lys Asn Arg Phe Leu Met Ser
       50                  55                  60
   Ala Glu Ala Phe Glu His Leu Lys Thr Gln Phe Asp Leu Asn Ala Glu
   65                  70                  75                  80
   Thr Asn Ala Cys Ser Ile Asn Gly Asn Ala Pro Ala Glu Ile Asp Leu
                   85                  90                  95
   Arg Gln Met Arg Thr Val Thr Pro Ile Arg Met Gln Gly Gly Cys Gly
               100                 105                 110
   Ser Cys Trp Ala Phe Ser Gly Val Ala Ala Thr Glu Ser Ala Tyr Leu
           115                 120                 125
   Ala Tyr Arg Asn Gln Ser Leu Asp Leu Ala Glu Gln Glu Leu Val Asp
       130                 135                 140
   Cys Ala Ser Gln His Gly Cys His Gly Asp Thr Ile Pro Arg Gly Ile
   145                 150                 155                 160
   Glu Tyr Ile Gln His Asn Gly Val Val Gln Glu Ser Tyr Tyr Arg Tyr
                   165                 170                 175
   Val Ala Arg Glu Gln Ser Cys Arg Arg Pro Asn Ala Gln Arg Phe Gly
               180                 185                 190
   Ile Ser Asn Tyr Cys Gln Ile Tyr Pro Pro Asn Ala Asn Lys Ile Arg
           195                 200                 205
   Glu Ala Leu Ala Gln Thr His Ser Ala Ile Ala Val Ile Ile Gly Ile
       210                 215                 220
   Lys Asp Leu Asp Ala Phe Arg His Tyr Asp Gly Arg Thr Ile Ile Gln
   225                 230                 235                 240
   Arg Asp Asn Gly Tyr Gln Pro Asn Tyr His Ala Val Asn Ile Val Gly
                   245                 250                 255
   Tyr Ser Asn Ala Gln Gly Val Asp Tyr Trp Ile Val Arg Asn Ser Trp
               260                 265                 270
   Asp Thr Asn Trp Gly Asp Asn Gly Tyr Gly Tyr Phe Ala Ala Asn Ile
           275                 280                 285
   Asp Leu Met Met Ile Glu Glu Tyr Pro Tyr Val Val Ile Leu
       290                 295                 300
<210> 8
   <211> 351
   <212> PRT
   <213> Artificial
<220>
   <223> Derp2-Derp1 fusion
<400> 8
   Asp Gln Val Asp Val Lys Asp Cys Ala Asn His Glu Ile Lys Lys Val
   1               5                   10                  15
   Leu Val Pro Gly Cys His Gly Ser Glu Pro Cys Ile Ile His Arg Gly
               20                  25                  30
   Lys Pro Phe Gln Leu Glu Ala Val Phe Glu Ala Asn Gln Asn Thr Lys
           35                  40                  45
   Thr Ala Lys Ile Glu Ile Lys Ala Ser Ile Asp Gly Leu Glu Val Asp
       50                  55                  60
   Val Pro Gly Ile Asp Pro Asn Ala Cys His Tyr Met Lys Cys Pro Leu
   65                  70                  75                  80
   Val Lys Gly Gln Gln Tyr Asp Ile Lys Tyr Thr Trp Asn Val Pro Lys
                   85                  90                  95
   Ile Ala Pro Lys Ser Glu Asn Val Val Val Thr Val Lys Val Met Gly
               100                 105                 110
   Asp Asp Gly Val Leu Ala Cys Ala Ile Ala Thr His Ala Lys Ile Arg
           115                 120                 125
   Asp Thr Asn Ala Cys Ser Ile Asn Gly Asn Ala Pro Ala Glu Ile Asp
       130                 135                 140
   Leu Arg Gln Met Arg Thr Val Thr Pro Ile Arg Met Gln Gly Gly Cys
   145                 150                 155                 160
   Gly Ser Cys Trp Ala Phe Ser Gly Val Ala Ala Thr Glu Ser Ala Tyr
   165 170 175
   Leu Ala Tyr Arg Asn Gln Ser Leu Asp Leu Ala Glu Gln Glu Leu Val
   180 185 190
   Asp Cys Ala Ser Gln His Gly Cys His Gly Asp Thr Ile Pro Arg Gly
   195 200 205
   Ile Glu Tyr Ile Gln His Asn Gly Val Val Gln Glu Ser Tyr Tyr Arg
   210 215 220
   Tyr Val Ala Arg Glu Gln Ser Cys Arg Arg Pro Asn Ala Gln Arg Phe
   225 230 235 240
   Gly Ile Ser Asn Tyr Cys Gln Ile Tyr Pro Pro Asn Ala Asn Lys Ile
   245 250 255
   Arg Glu Ala Leu Ala Gln Thr His Ser Ala Ile Ala Val Ile Ile Gly
   260 265 270
   Ile Lys Asp Leu Asp Ala Phe Arg His Tyr Asp Gly Arg Thr Ile Ile
   275 280 285
   Gln Arg Asp Asn Gly Tyr Gln Pro Asn Tyr His Ala Val Asn Ile Val
   290 295 300
   Gly Tyr Ser Asn Ala Gln Gly Val Asp Tyr Trp Ile Val Arg Asn Ser
   305 310 315 320
   Trp Asp Thr Asn Trp Gly Asp Asn Gly Tyr Gly Tyr Phe Ala Ala Asn
   325 330 335
   Ile Asp Leu Met Met Ile Glu Glu Tyr Pro Tyr Val Val Ile Leu
   340 345 350
<210> 9
   <211> 431
   <212> PRT
   <213> Artificial
<220>
   <223> Derp2-ProDerp1 fusion
<400> 9
   Asp Gln Val Asp Val Lys Asp Cys Ala Asn His Glu Ile Lys Lys Val
   1               5                   10                  15
   Leu Val Pro Gly Cys His Gly Ser Glu Pro Cys Ile Ile His Arg Gly
               20                  25                  30
   Lys Pro Phe Gln Leu Glu Ala Val Phe Glu Ala Asn Gln Asn Thr Lys
           35                  40                  45
   Thr Ala Lys Ile Glu Ile Lys Ala Ser Ile Asp Gly Leu Glu Val Asp
       50                  55                  60
   Val Pro Gly Ile Asp Pro Asn Ala Cys His Tyr Met Lys Cys Pro Leu
   65                  70                  75                  80
   Val Lys Gly Gln Gln Tyr Asp Ile Lys Tyr Thr Trp Asn Val Pro Lys
                   85                  90                  95
   Ile Ala Pro Lys Ser Glu Asn Val Val Val Thr Val Lys Val Met Gly
               100                 105                 110
   Asp Asp Gly Val Leu Ala Cys Ala Ile Ala Thr His Ala Lys Ile Arg
           115                 120                 125
   Asp Arg Pro Ser Ser Ile Lys Thr Phe Glu Glu Tyr Lys Lys Ala Phe
       130                 135                 140
   Asn Lys Ser Tyr Ala Thr Phe Glu Asp Glu Glu Ala Ala Arg Lys Asn
   145                 150                 155                 160
   Phe Leu Glu Ser Val Lys Tyr Val Gln Ser Asn Gly Gly Ala Ile Asn
                   165                 170                 175
   His Leu Ser Asp Leu Ser Leu Asp Glu Phe Lys Asn Arg Phe Leu Met
               180                 185                 190
   Ser Ala Glu Ala Phe Glu His Leu Lys Thr Gln Phe Asp Leu Asn Ala
           195                 200                 205
   Glu Thr Asn Ala Cys Ser Ile Asn Gly Asn Ala Pro Ala Glu Ile Asp
       210                 215                 220
   Leu Arg Gln Met Arg Thr Val Thr Pro Ile Arg Met Gln Gly Gly Cys
   225                 230                 235                 240
   Gly Ser Cys Trp Ala Phe Ser Gly Val Ala Ala Thr Glu Ser Ala Tyr
                   245                 250                 255
   Leu Ala Tyr Arg Asn Gln Ser Leu Asp Leu Ala Glu Gln Glu Leu Val
   260 265 270
   Asp Cys Ala Ser Gln His Gly Cys His Gly Asp Thr Ile Pro Arg Gly
   275 280 285
   Ile Glu Tyr Ile Gln His Asn Gly Val Val Gln Glu Ser Tyr Tyr Arg
   290 295 300
   Tyr Val Ala Arg Glu Gln Ser Cys Arg Arg Pro Asn Ala Gln Arg Phe
   305 310 315 320
   Gly Ile Ser Asn Tyr Cys Gln Ile Tyr Pro Pro Asn Ala Asn Lys Ile
   325 330 335
   Arg Glu Ala Leu Ala Gln Thr His Ser Ala Ile Ala Val Ile Ile Gly
   340 345 350
   Ile Lys Asp Leu Asp Ala Phe Arg His Tyr Asp Gly Arg Thr Ile Ile
   355 360 365
   Gln Arg Asp Asn Gly Tyr Gln Pro Asn Tyr His Ala Val Asn Ile Val
   370 375 380
   Gly Tyr Ser Asn Ala Gln Gly Val Asp Tyr Trp Ile Val Arg Asn Ser
   385 390 395 400
   Trp Asp Thr Asn Trp Gly Asp Asn Gly Tyr Gly Tyr Phe Ala Ala Asn
   405 410 415
   Ile Asp Leu Met Met Ile Glu Glu Tyr Pro Tyr Val Val Ile Leu
   420 425 430
<210> 10
   <211> 351
   <212> PRT
   <213> Artificial
<220>
   <223> Derp1-Derp2 fusion
<400> 10
   Thr Asn Ala Cys Ser Ile Asn Gly Asn Ala Pro Ala Glu Ile Asp Leu
   1 5 10 15
   Arg Gln Met Arg Thr Val Thr Pro Ile Arg Met Gln Gly Gly Cys Gly
               20                      25                      30
   Ser Cys Trp Ala Phe Ser Gly Val Ala Ala Thr Glu Ser Ala Tyr Leu
           35                  40                      45
   Ala Tyr Arg Asn Gln Ser Leu Asp Leu Ala Glu Gln Glu Leu Val Asp
       50                  55                      60
   Cys Ala Ser Gln His Gly Cys His Gly Asp Thr Ile Pro Arg Gly Ile
   65                  70                      75                  80
   Glu Tyr Ile Gln His Asn Gly Val Val Gln Glu Ser Tyr Tyr Arg Tyr
                   85                      90                  95
   Val Ala Arg Glu Gln Ser Cys Arg Arg Pro Asn Ala Gln Arg Phe Gly
               100                     105                 110
   Ile Ser Asn Tyr Cys Gln Ile Tyr Pro Pro Asn Ala Asn Lys Ile Arg
           115                     120                 125
   Glu Ala Leu Ala Gln Thr His Ser Ala Ile Ala Val Ile Ile Gly Ile
       130                     135                 140
   Lys Asp Leu Asp Ala Phe Arg His Tyr Asp Gly Arg Thr Ile Ile Gln
   145                     150                 155                 160
   Arg Asp Asn Gly Tyr Gln Pro Asn Tyr His Ala Val Asn Ile Val Gly
                   165                 170                     175
   Tyr Ser Asn Ala Gln Gly Val Asp Tyr Trp Ile Val Arg Asn Ser Trp
               180                     185                 190
   Asp Thr Asn Trp Gly Asp Asn Gly Tyr Gly Tyr Phe Ala Ala Asn Ile
           195                     200                 205
   Asp Leu Met Met Ile Glu Glu Tyr Pro Tyr Val Val Ile Leu Asp Gln
       210                     215                 220
   Val Asp Val Lys Asp Cys Ala Asn His Glu Ile Lys Lys Val Leu Val
   225                     230                 235                 240
   Pro Gly Cys His Gly Ser Glu Pro Cys Ile Ile His Arg Gly Lys Pro
                   245                 250                     255
   Phe Gln Leu Glu Ala Val Phe Glu Ala Asn Gln Asn Thr Lys Thr Ala
               260                     265                 270
   Lys Ile Glu Ile Lys Ala Ser Ile Asp Gly Leu Glu Val Asp Val Pro
           275                 280                 285
   Gly Ile Asp Pro Asn Ala Cys His Tyr Met Lys Cys Pro Leu Val Lys
       290                 295                 300
   Gly Gln Gln Tyr Asp Ile Lys Tyr Thr Trp Asn Val Pro Lys Ile Ala
   305                 310                 315                     320
   Pro Lys Ser Glu Asn Val Val Val Thr Val Lys Val Met Gly Asp Asp
                   325                 330                 335
   Gly Val Leu Ala Cys Ala Ile Ala Thr His Ala Lys Ile Arg Asp
               340                 345                 350
<210> 11
   <211> 431
   <212> PRT
   <213> Artificial
<220>
   <223> ProDerp1-Derp2
<400> 11
   Arg Pro Ser Ser Ile Lys Thr Phe Glu Glu Tyr Lys Lys Ala Phe Asn
   1               5                   10                  15
   Lys Ser Tyr Ala Thr Phe Glu Asp Glu Glu Ala Ala Arg Lys Asn Phe
               20                  25                  30
   Leu Glu Ser Val Lys Tyr Val Gln Ser Asn Gly Gly Ala Ile Asn His
           35                  40                  45
   Leu Ser Asp Leu Ser Leu Asp Glu Phe Lys Asn Arg Phe Leu Met Ser
       50                  55                  60
   Ala Glu Ala Phe Glu His Leu Lys Thr Gln Phe Asp Leu Asn Ala Glu
   65                  70                  75                  80
   Thr Asn Ala Cys Ser Ile Asn Gly Asn Ala Pro Ala Glu Ile Asp Leu
                   85                  90                  95
   Arg Gln Met Arg Thr Val Thr Pro Ile Arg Met Gln Gly Gly Cys Gly
               100                 105                 110
   Ser Cys Trp Ala Phe Ser Gly Val Ala Ala Thr Glu Ser Ala Tyr Leu
   115 120 125
   Ala Tyr Arg Asn Gln Ser Leu Asp Leu Ala Glu Gln Glu Leu Val Asp
   130 135 140
   Cys Ala Ser Gln His Gly Cys His Gly Asp Thr Ile Pro Arg Gly Ile
   145 150 155 160
   Glu Tyr Ile Gln His Asn Gly Val Val Gln Glu Ser Tyr Tyr Arg Tyr
   165 170 175
   Val Ala Arg Glu Gln Ser Cys Arg Arg Pro Asn Ala Gln Arg Phe Gly
   180 185 190
   Ile Ser Asn Tyr Cys Gln Ile Tyr Pro Pro Asn Ala Asn Lys Ile Arg
   195 200 205
   Glu Ala Leu Ala Gln Thr His Ser Ala Ile Ala Val Ile Ile Gly Ile
   210 215 220
   Lys Asp Leu Asp Ala Phe Arg His Tyr Asp Gly Arg Thr Ile Ile Gln
   225 230 235 240
   Arg Asp Asn Gly Tyr Gln Pro Asn Tyr His Ala Val Asn Ile Val Gly
   245 250 255
   Tyr Ser Asn Ala Gln Gly Val Asp Tyr Trp Ile Val Arg Asn Ser Trp
   260 265 270
   Asp Thr Asn Trp Gly Asp Asn Gly Tyr Gly Tyr Phe Ala Ala Asn Ile
   275 280 285
   Asp Leu Met Met Ile Glu Glu Tyr Pro Tyr Val Val Ile Leu Asp Gln
   290 295 300
   Val Asp Val Lys Asp Cys Ala Asn His Glu Ile Lys Lys Val Leu Val
   305 310 315 320
   Pro Gly Cys His Gly Ser Glu Pro Cys Ile Ile His Arg Gly Lys Pro
   325 330 335
   Phe Gln Leu Glu Ala Val Phe Glu Ala Asn Gln Asn Thr Lys Thr Ala
   340 345 350
   Lys Ile Glu Ile Lys Ala Ser Ile Asp Gly Leu Glu Val Asp Val Pro
   355 360 365
   Gly Ile Asp Pro Asn Ala Cys His Tyr Met Lys Cys Pro Leu Val Lys
       370                 375                 380
   Gly Gln Gln Tyr Asp Ile Lys Tyr Thr Trp Asn Val Pro Lys Ile Ala
   385                 390                 395                 400
   Pro Lys Ser Glu Asn Val Val Val Thr Val Lys Val Met Gly Asp Asp
                   405                 410                 415
   Gly Val Leu Ala Cys Ala Ile Ala Thr His Ala Lys Ile Arg Asp
               420                 425                 430
<210> 12
   <211> 1053
   <212> DNA
   <213> Artificial
<220>
   <223> Derp2-Derp1 DNA
<400> 12
   gatcaagtcg atgtcaaaga ttgtgccaat catgaaatca aaaaagtttt ggtaccagga     60
   tgccatggtt cagaaccatg tatcattcat cgtggtaaac cattccaatt ggaagccgtt    120
   ttcgaagcca accaaaacac aaaaacggct aaaattgaaa tcaaagcctc aatcgatggt    180
   ttagaagttg atgttcccgg tatcgatcca aatgcatgcc attacatgaa atgcccattg    240
   gttaaaggac aacaatatga tattaaatat acatggaatg ttccgaaaat tgcaccaaaa    300
   tctgaaaatg ttgtcgtcac tgttaaagtt atgggtgatg atggtgtttt ggcctgtgct    360
   attgctactc atgctaaaat ccgcgatact aacgcctgca gtatcaatgg aaatgctcca    420
   gctgaaatcg atttgcgaca aatgcgaact gtcactccca ttcgtatgca aggaggctgt    480
   ggttcatgtt gggctttctc tggtgttgcc gcaactgaat cagcttattt ggcttaccgt    540
   aatcaatcat tggatcttgc tgaacaagaa ttagtcgatt gtgcttccca acacggttgt    600
   catggtgata ccattccacg tggtattgaa tacatccaac ataatggtgt cgtccaagaa    660
   agctactatc gatacgttgc acgagaacaa tcatgccgac gaccaaatgc acaacgtttc    720
   ggtatctcaa actattgcca aatttaccca ccaaatgcaa acaaaattcg tgaagctttg    780
   gctcaaaccc acagcgctat tgccgtcatt attggcatca aagatttaga cgcattccgt    840
   cattatgatg gccgaacaat cattcaacgc gataatggtt accaaccaaa ctatcacgct    900
   gtcaacattg ttggttacag taacgcacaa ggtgtcgatt attggatcgt acgaaacagt    960
   tgggatacca attggggtga taatggttac ggttattttg ctgccaacat cgatttgatg   1020
   atgattgaag aatatccata tgttgtcatt ctc 1053
<210> 13
   <211> 1293
   <212> DNA
   <213> Artificial
<220>
   <223> Derp2-ProDerp1 DNA
<400> 13
   gatcaagtcg atgtcaaaga ttgtgccaat catgaaatca aaaaagtttt ggtaccagga 60
   tgccatggtt cagaaccatg tatcattcat cgtggtaaac cattccaatt ggaagccgtt 120
   ttcgaagcca accaaaacac aaaaacggct aaaattgaaa tcaaagcctc aatcgatggt 180
   ttagaagttg atgttcccgg tatcgatcca aatgcatgcc attacatgaa atgcccattg 240
   gttaaaggac aacaatatga tattaaatat acatggaatg ttccgaaaat tgcaccaaaa 300
   tctgaaaatg ttgtcgtcac tgttaaagtt atgggtgatg atggtgtttt ggcctgtgct 360
   attgctactc atgctaaaat ccgcgatcgt ccatcatcga tcaaaacttt tgaagaatac 420
   aaaaaagcct tcaacaaaag ttatgctacc ttcgaagatg aagaagctgc ccgtaaaaac 480
   tttttggaat cagtaaaata tgttcaatca aatggaggtg ccatcaacca tttgtccgat 540
   ttgtcgttgg atgaattcaa aaaccgattt ttgatgagtg cagaagcttt tgaacacctc 600
   aaaactcaat tcgatttgaa tgctgaaact aacgcctgca gtatcaatgg aaatgctcca 660
   gctgaaatcg atttgcgaca aatgcgaact gtcactccca ttcgtatgca aggaggctgt 720
   ggttcatgtt gggctttctc tggtgttgcc gcaactgaat cagcttattt ggcttaccgt 780
   aatcaatcat tggatcttgc tgaacaagaa ttagtcgatt gtgcttccca acacggttgt 840
   catggtgata ccattccacg tggtattgaa tacatccaac ataatggtgt cgtccaagaa 900
   agctactatc gatacgttgc acgagaacaa tcatgccgac gaccaaatgc acaacgtttc 960
   ggtatctcaa actattgcca aatttaccca ccaaatgcaa acaaaattcg tgaagctttg 1020
   gctcaaaccc acagcgctat tgccgtcatt attggcatca aagatttaga cgcattccgt 1080
   cattatgatg gccgaacaat cattcaacgc gataatggtt accaaccaaa ctatcacgct 1140
   gtcaacattg ttggttacag taacgcacaa ggtgtcgatt attggatcgt acgaaacagt 1200
   tgggatacca attggggtga taatggttac ggttattttg ctgccaacat cgatttgatg 1260
   atgattgaag aatatccata tgttgtcatt ctc 1293
<210> 14
   <211> 1053
   <212> DNA
   <213> Artificial
<220>
   <223> Derp1-Derp2 DNA
<400> 14
   actaacgcct gcagtatcaa tggaaatgct ccagctgaaa tcgatttgcg acaaatgcga     60
   actgtcactc ccattcgtat gcaaggaggc tgtggttcat gttgggcttt ctctggtgtt    120
   gccgcaactg aatcagctta tttggcttac cgtaatcaat cattggatct tgctgaacaa    180
   gaattagtcg attgtgcttc ccaacacggt tgtcatggtg ataccattcc acgtggtatt    240
   gaatacatcc aacataatgg tgtcgtccaa gaaagctact atcgatacgt tgcacgagaa    300
   caatcatgcc gacgaccaaa tgcacaacgt ttcggtatct caaactattg ccaaatttac    360
   ccaccaaatg caaacaaaat tcgtgaagct ttggctcaaa cccacagcgc tattgccgtc    420
   attattggca tcaaagattt agacgcattc cgtcattatg atggccgaac aatcattcaa    480
   cgcgataatg gttaccaacc aaactatcac gctgtcaaca ttgttggtta cagtaacgca    540
   caaggtgtcg attattggat cgtacgaaac agttgggata ccaattgggg tgataatggt    600
   tacggttatt ttgctgccaa catcgatttg atgatgattg aagaatatcc atatgttgtc    660
   attctcgatc aagtcgatgt caaagattgt gccaatcatg aaatcaaaaa agttttggta    720
   ccaggatgcc atggttcaga accatgtatc attcatcgtg gtaaaccatt ccaattggaa    780
   gccgttttcg aagccaacca aaacacaaaa acggctaaaa ttgaaatcaa agcctcaatc    840
   gatggtttag aagttgatgt tcccggtatc gatccaaatg catgccatta catgaaatgc    900
   ccattggtta aaggacaaca atatgatatt aaatatacat ggaatgttcc gaaaattgca    960
   ccaaaatctg aaaatgttgt cgtcactgtt aaagttatgg gtgatgatgg tgttttggcc   1020
   tgtgctattg ctactcatgc taaaatccgc gat                                1053
<210> 15
   <211> 1293
   <212> DNA
   <213> Artificial
<220>
   <223> ProDerp1-Derp2 DNA
<400> 15
   cgtccatcat cgatcaaaac ttttgaagaa tacaaaaaag ccttcaacaa aagttatgct      60
   accttcgaag atgaagaagc tgcccgtaaa aactttttgg aatcagtaaa atatgttcaa     120
   tcaaatggag gtgccatcaa ccatttgtcc gatttgtcgt tggatgaatt caaaaaccga     180
   tttttgatga gtgcagaagc ttttgaacac ctcaaaactc aattcgattt gaatgctgaa     240
   actaacgcct gcagtatcaa tggaaatgct ccagctgaaa tcgatttgcg acaaatgcga     300
   actgtcactc ccattcgtat gcaaggaggc tgtggttcat gttgggcttt ctctggtgtt     360
   gccgcaactg aatcagctta tttggcttac cgtaatcaat cattggatct tgctgaacaa     420
   gaattagtcg attgtgcttc ccaacacggt tgtcatggtg ataccattcc acgtggtatt     480
   gaatacatcc aacataatgg tgtcgtccaa gaaagctact atcgatacgt tgcacgagaa     540
   caatcatgcc gacgaccaaa tgcacaacgt ttcggtatct caaactattg ccaaatttac     600
   ccaccaaatg caaacaaaat tcgtgaagct ttggctcaaa cccacagcgc tattgccgtc     660
   attattggca tcaaagattt agacgcattc cgtcattatg atggccgaac aatcattcaa     720
   cgcgataatg gttaccaacc aaactatcac gctgtcaaca ttgttggtta cagtaacgca     780
   caaggtgtcg attattggat cgtacgaaac agttgggata ccaattgggg tgataatggt     840
   tacggttatt ttgctgccaa catcgatttg atgatgattg aagaatatcc atatgttgtc     900
   attctcgatc aagtcgatgt caaagattgt gccaatcatg aaatcaaaaa agttttggta     960
   ccaggatgcc atggttcaga accatgtatc attcatcgtg gtaaaccatt ccaattggaa    1020
   gccgttttcg aagccaacca aaacacaaaa acggctaaaa ttgaaatcaa agcctcaatc    1080
   gatggtttag aagttgatgt tcccggtatc gatccaaatg catgccatta catgaaatgc    1140
   ccattggtta aaggacaaca atatgatatt aaatatacat ggaatgttcc gaaaattgca    1200
   ccaaaatctg aaaatgttgt cgtcactgtt aaagttatgg gtgatgatgg tgttttggcc    1260
   tgtgctattg ctactcatgc taaaatccgc gat                                  1293
<210> 16
   <211> 36
   <212> DNA
   <213> Artificial
<220>
   <223> primer
<400> 16
   ggttgctctt ccaacgatca agtcgatgtc aaagat 36
<210> 17
   <211> 37
   <212> DNA
   <213> Artificial
<220>
   <223> primer
<400> 17
   catgctaaaa tccgcgatac ttaacgcctg cagtatc 37
<210> 18
   <211> 36
   <212> DNA
   <213> Artificial
<220>
   <223> primer
<400> 18
   gatactgcag gcgttagtat cgcggatttt agcatg 36
<210> 19
   <211> 32
   <212> DNA
   <213> Artificial
<220>
   <223> primer
<400> 19
   gcggccgctc agagaatgac aacatatgga ta 32
<210> 20
   <211> 36
   <212> DNA
   <213> Artificial
<220>
   <223> primer
<400> 20
   gggctcgaga aaagagatca agtcgatgtc aaagat 36
<210> 21
   <211> 33
   <212> DNA
   <213> Artificial
<220>
   <223> primer
<400> 21
   ggcggccgct cagagaatga caacatatgg ata 33
<210> 22
   <211> 36
   <212> DNA
   <213> Artificial
<220>
   <223> primer
<400> 22
   catgctaaaa tccgcgatcg tccatcatcg atcaaa 36
<210> 23
   <211> 36
   <212> DNA
   <213> Artificial
<220>
   <223> primer
<400> 23
   tttgatcgat gatggacgat cgcggatttt agcatg 36
<210> 24
   <211> 33
   <212> DNA
   <213> Artificial
<220>
   <223> primer
<400> 24
   gggctcgaga aaagaactaa cgcctgcagt atc 33
<210> 25
   <211> 36
   <212> DNA
   <213> Artificial
<220>
   <223> primer
<400> 25
   ccatatgttg tcattctcga tcaagtcgat gtcaaa 36
<210> 26
   <211> 36
   <212> DNA
   <213> Artificial
<220>
   <223> primer
<400> 26
   tttgacatcg acttgatcga gaatgacaac atatgg 36
<210> 27
   <211> 33
   <212> DNA
   <213> Artificial
<220>
   <223> primer
<400> 27
   ggcggccgct caatcgcgga ttttagcatg agt 33
<210> 28
   <211> 36
   <212> DNA
   <213> Artificial
<220>
   <223> primer
<400> 28
   gggctcgaga aaagacgtcc atcatcgatc aaaact 36
<210> 29
   <211> 41
   <212> DNA
   <213> Artificial
<220>
   <223> primer
<400> 29
   atcaccatca ccacggtatg caagatcaag tcgatgtcaa a 41
<210> 30
   <211> 40
   <212> DNA
   <213> Artificial
<220>
   <223> primer
<400> 30
   cttggttagt tagttattag agaatgacaa catatggata 40
<210> 31
   <211> 41
   <212> DNA
   <213> Artificial
<220>
   <223> primer
<400> 31
   atcaccatca ccacggtatg caaactaacg cctgcagtat c 41
<210> 32
   <211> 40
   <212> DNA
   <213> Artificial
<220>
   <223> primer
<400> 32
   cttggttagt tagttattaa tcgcggattt tagcatgagt 40
<210> 33
   <211> 41
   <212> DNA
   <213> Artificial
<220>
   <223> primer
<400> 33
   atcaccatca ccacggtatg caacgtccat catcgatcaa a 41